# EUROPEAN PATENT SPECIFICATION

(11) **EP 1 773 994 B1**
(45) Date of publication and mention of the grant of the patent: **05.11.2008**
(21) Application number: 05770534.5
(22) Date of filing: 26.07.2005
(51) Int. Cl.: C12N 15/10

(54) **POLYPEPTIDE**
POLYPEPTID
POLYPEPTIDE

(30) Priority: 26.07.2004 GB 0416651
(43) Date of publication of application: 18.04.2007
(73) Proprietor: Bioneer A/S, 2970 Hoersholm (DK)
(72) Inventor: KRISTENSEN, Peter, DK-8310 Tranbjerg (DK)
(74) Representative: Dempster, Robert Charles
(86) International application number: PCT/IB2005/002530
(87) International publication number: WO 2006/013468

(56) References cited:
- WO-A-01/09177
- GUNNARSSON L CICORTAS ET AL: "A carbohydrate binding module as a diversity-carrying scaffold" PROTEIN ENGINEERING DESIGN & SELECTION, vol. 17, no. 3, March 2004 (2004-03), pages 213-221, XP002381837 ISSN: 1741-0126
- SIMPSON PETER J ET AL: "The solution structure of the CBM4-2 carbohydrate binding module from a thermostable Rhodothermus marinus xylanase" BIOCHEMISTRY, vol. 41, no. 18, 7 May 2002 (2002-05-07), pages 5712-5719, XP002381838 ISSN: 0006-2960
- PROBA K ET AL: "Antibody scFv fragments without disulfide bonds made by molecular evolution" JOURNAL OF MOLECULAR BIOLOGY, LONDON, GB, vol. 275, no. 2, 16 January 1998 (1998-01-16), pages 245-253, XP002255006 ISSN: 0022-2836
- A. KOIDE ET AL.: "The fibronectin type III domain as a scaffold for novel binding proteins." JOURNAL OF MOLECULAR BIOLOGY, vol. 284, no. 4, 11 December 1998 (1998-12-11), pages 1141-1151, XP002944100 London, GB
- NYGREN P-A ET AL: "Binding proteins from alternative scaffolds" JOURNAL OF IMMUNOLOGICAL METHODS, ELSEVIER SCIENCE PUBLISHERS B.V.,AMSTERDAM, NL, vol. 290, no. 1-2, July 2004 (2004-07), pages 3-28, XP004521978 ISSN: 0022-1759
- BINZ H KASPAR ET AL: "Engineering novel binding proteins from nonimmunoglobulin domains" NATURE BIOTECHNOLOGY, vol. 23, no. 10, October 2005 (2005-10), pages 1257-1268, XP002381839 ISSN: 1087-0156

## Description

The present invention relates to a novel polypeptide chain forming a particular 3 dimensional fold characterised by a β-sandwich. By altering the amino acid sequence in specific regions of the polypeptide of the invention novel binding characteristics are developed. Methods for the generation of a polypeptide according to the invention and uses of polypeptides of the invention are also described.

### BACKGROUND OF THE INVENTION.

Macromolecular recognition takes place when two or more surfaces are capable of establishing sufficient points of contacts, allowing specific binding to occur. Although macromolecular recognition is a generally observed phenomenon in all living systems, the best characterised has been the immune system. One example being the human immune system, consisting of a large number of structural similar antibody molecules, each having minor sequence differences in the variable regions of the antibody structure. These small difference result in the generation of a pletphora of binding specificities.

Naturally occurring antibodies are multi-domain proteins composed of heavy and light chains. The overall antibody structure can be divided into different functional domains according to their biological function. Certain parts of the antibody molecule interacts with receptor molecules on the eukaryotic cells, thus giving rise to a biological response, these parts are often called constant domains. Other parts of the antibody molecule contain a large degree of sequence variation between different antibody molecules, thus providing different antibody molecules that ability to bind to a divers range of different molecules. The sequence variation observed in the variable parts of the antibody molecule allows an immense number of different structural surfaces to be created. When the variable region of a given antibody can make enough interactions with an antigen specific binding occur. The sequence variation of different antibodies are confined to six loop regions, three on the heavy chain and three on the light chain.

These loop regions are carried on a so called β sandwich structure, where the individual loops connect β strands (Amit AG. 1986).

It has previously been demonstrated that smaller fragments of the antibody structure retain their antigen recognition properties. At the same time such smaller fragment may provide better reagents in certain therapeutic application. Most notably generation of fragments of the variable parts have been demonstrated, giving rice to either scFv fragments in which the variable parts of the heavy and light chain are linked by a peptide linker or artificial engineering of a disulphide bridge for covalent linking the heavy and light chain.

Recombinant DNA technology has provided tools which allows the in vitro generation of repertoires of antibody molecules. When introducing the genes encoding such antibodies in systems allowing a linkage of genotype with phenotype, selection of specific antibodies becomes feasible based on their binding characteristics (Marks JD et al. 1991). Most often such a system has been the phage display system (Smith GP. 1985).

The generation and selection of antibody fragments have been demonstrated to be a powerful technique for identifying novel binding molecules. However, such antibody fragments are composed of two chains (heavy and light) which have to fold together in order for a functional representation of the loop segments for antigen binding. Often functional folding of these two chains and their functional pairing is impaired, thus providing an unstable antibody fragment. Further a number of intra-chain disulfide bridges have to be formed to ensure the functional folding of such antibody fragments, thus limiting applications in which intracellular expression is an issue. Overall antibody fragments suffer from a series of serious drawbacks.

A number of novel scaffolds have been described, all of which have the common feature that random sequences can be accommadated, examples being the development of the Z domain (Nord K. et al. 1997), minibody (Pessi A. et al. 1995), Tendamistat (McConnel SJ. And Hoess RH 1995), zinc finger (Choo Y. and Klug A. 1995), cytochrome B₅₆₂ (Ku J. and Schults P.G. 1995), trypsin inhibitor (Rottgen P. and Collins J. 1995), synthetic colied coil (Houston M.E. et al. 1996), knottins (Smith G.P. et al. 1998), green fluorescent protein (Abedi M.R. et al 1998), fibronectin (Koide A. et al. 1998), anticalin (Beste G. et al. 1999), CTLA-4 (Nuttall S.D. et al. 1999) and tetranectin (Graversen J.H. et al. 2000). Each of these scaffold have certain unique features which make the application in defined areas beneficial, but as with antibodies each also has several drawbacks. The following invention presents an optimized scaffold, generated to solve some of the above mentioned problems, such as the application to intracellular ligand binding.

Fatty Acid Binding Proteins (FABP) are a diverse family of intracellular proteins. The precise physiological role of FABP is not fully understood, but accumulating evidence suggests that the main function of the FABPs are to bind fatty acids, which exhibits limited solubility, and thereby aid transportation of fatty acids (Stewart J.M. 2000).

An adequate supply of long-chain fatty acids is important for proper functioning of eukaryotic cells. Fatty acids act as building blocks for membrane phospholipids and are a main substrate for energy production. In addition fatty acids are precursors of signaling molecules and mediators of the expression of various genes encoding proteins involved in lipid metabolism. The binding characteristics of H-FABP and its predominant presence in types I and IIA muscle fibers already suggests its functional involvement in oxidative metabolism (Glatz JFC 2003)

The intracellular and cytoplasmic FABPs form a group of at least nine distinct protein types. They are 14- to 15-kDa proteins of 126-134 amino acids, and are named after the first tissue of isolation or identification (Table 1)(Zimmerman A.W. 2001). Some types (L-FABP, H-FABP) occur in more than one tissue whereas others (I-FABP, A-FABP, M-FABP, B-FABP) are limited to only one tissue.

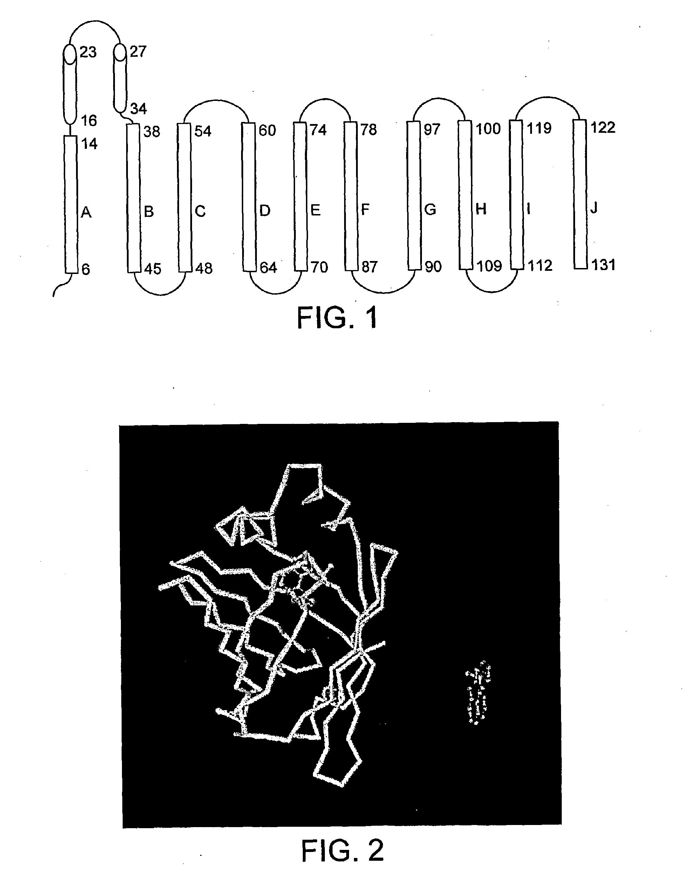

The first reported crystallographic studies to enter the literature were of recombinant rat I-FABP (Sacchettini J.C. et al. 1988). Structural analyses of several FABPs have revealed markedly similar three-dimensional folds consisting of 10 antiparallel β-strands that form a β-barrel. This β-barrel is capped by two short α-helices arranged in a helix-loop-helix structure (figure 1). The present evidence suggests that the helix-loop-helix structure together with the turns connecting β-strands C-D and D-E, functions as a "dynamic portal" that regulates Fatty Acid entry and exit from the internal ligand binding cavity (Reese-Wagoner A. et al. 1999). In particular the transfer of Fatty Acids to membranes seems to be controlled by the helix-loop-helix motif (Liou H-L et al 2002, Córsico B 2004)). This class of FABP's, to which H-FABP also belongs, has been termed membrane-active FABPs. These catalyses both the dissociation of the fatty acid from the donor membrane and binding to the acceptor membrane (Glatz J.F.C. 2003).

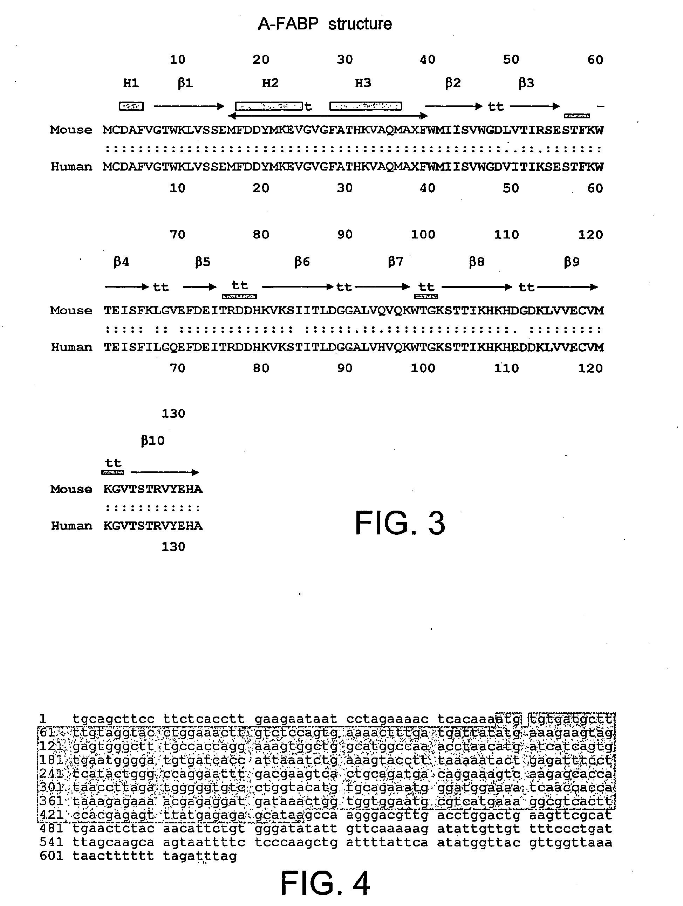

The topology of the FABP's is comparable to two other families of closely related structural families, namely the Lipocalins and the streptavidins. However whereas the structure of the FABP family comprises a 10 β-sheet clam structure with a helix-loop-helix lid, the two others comprise structures of 8 β-strand and no helix-loop-helix motif. The FABP barrel is more flattened and elliptical than either that of lipocalins or streptavidin (figure 2 and figure 3). Based on the structural similarities the three distinct families has been suggested to form part of a larger group, the calycin structural superfamily (Flower D.R. 1993). In contrast to the remarkably similar structures, the members of the FABP family show an amino acid sequence similarity of 22-73 %, with 39 highly conserved residues (Zimmerman A.W. and Veerkamp J.H. 2002).

The presence of one or more disulphide bridges is a unique feature of the E-FABP, whereas the cystein residues present in the other FABP molecules has been shown not to participate in disulphide bridge formation.

In general proteins of the lipocalin and streptavidin families are extracellular proteins, whereas the FABP's are intracellular. Due to the cellular distribution most FABP's do not contain cysteins which can participate in disulphide bridge formation, the one exception being Epidermal-FABP (Gutierrez-Gonzalez L.H. et al. 2002), although formation of a functional disulphide bridge has not been verified. Lipocalins and streptavidins on the other hand most often rely on disulphide bridge formation for structural stability (Flower D.R 1996)

In addition to binding of Fatty Acids in the cavity of FABP's, probes such as bisANS and ANS has been shown to bind, such binding usually has been associated with properties of a molten globule state of a protein, consequently indicating a flexible structure with a high degree of lose structure. However for I-FABP the binding of bisANS and ANS was verified to take place inside the pocket of this compact well-folded structure (Arighi C.N. et al 2003). They all possess a ligand binding cavity of approximately 1000 Å2 located in the top half of the β-barrel.

Gunnarsson et al (2004), Protein Engineering Design and Selection 17(3): 213-221 discloses a carbohydrate binding molecule (CBM4-2) as a scaffold.

Simpson et al (2002), Biochemistry 41(18): 5712-5719 discloses a solution structure of the CBM4-2 binding molecule. Proba et al (1998), Journal of Molecular Biology 275(2): 245-253 discloses scFv antibody fragments which do not require disulphide bonds for their stability.

### SUMMARY OF THE INVENTION.

The present invention relates to a novel polypeptide chain forming a particular 3 dimensional fold characterised by a β-sandwich. By altering the amino acid sequence in specific regions of the polypeptide of the invention the specificity of ligand binding can be adjusted.

Thus in a first aspect the present invention provides a fatty acid binding protein scaffold (FASTbody) capable of specific binding to one or more ligands, which scaffold comprises a single-chain polypeptide with the following structural properties:
(a) The scaffold contains 10 β-stands (designated ABCDEFGHI and J) connected by loop regions which determine the specificity of ligand binding, wherein the β-strands together form a β-clam structure; and wherein
(b) The loop regions connecting β-A and β -B; β -C and β -D; β -E and β -F; β -G and β -H; β-I and β-J are located on the same site of the β-clam structure;
wherein the fatty acid binding scaffold does not contain any disulphide bridge forming cysteines and wherein the scaffold does not comprise a helix-loop-helix motif.

In the FASTbodies described above, specific ligand binding takes place via the interaction of one or more loops regions of the fatty acid binding protein scaffold (FASTbody) with that specific ligand.

According to the above aspect of the invention, advantageously, the FASTbody according to the invention is capable of specific binding to RAS.

According to the above aspect of the invention, advantageously, a FASTbody according to the present invention consists of a single-chain polypeptide with the following structural properties:
(a) The scaffold contains 10 β-strands (designated ABCDEFGHI and J) connected by loop regions which determine the specificity of ligand binding, wherein the β-stands together form a β-clam structure; and wherein
(b) The loop regions connecting β-A and β -B; β -C and β -D; β -E and β -F; β -G and β -H; β-I and β-J are located on the same site of the β-clam structure; wherein the fatty acid binding scaffold does not contain any disulphide bridge forming cysteines; wherein the scaffold does not comprise a helix-loop-helix motif.

According to the above aspect of the invention, preferably the helix-loop-helix motif of a fatty acid binding protein is replaced with another peptide during the generation of the FASTbodies of the invention. Such a peptide may be of any suitable length and sequence. Advantageously, the peptide is a 9 amino acid random peptide. Most preferably, the 9 amino acid random peptide is as herein described.

According to the above aspect of the invention, the term 'a fatty acid binding protein scaffold' (FASTbody) refers to a scaffold according to the invention as described above. The inventors have devised the essential features of the ligand binding scaffold according to the invention based on experiments designed to investigate the structure/function relationships in various fatty acid binding proteins. A selection of these experiments are described in the Examples. In particular, the fatty acid binding scaffold according to the invention was initially generated by removing the helix-loop-helix motif from adipocyte fatty acid binding protein (A-FABP) and replacing it with a randomised 9 amino-acid peptide, in particular a 9 amino-acid random peptide as described herein.

In a further preferred embodiment of the invention, the fatty acid binding protein scaffold according to the invention comprises, preferably consists of a fatty acid binding protein FABP in which the helix-loop-helix motif has been replaced with an amino acid sequence. Preferably, the helix-loop-helix motif has been replaced with a random amino acid sequence as herein defined. Most preferably, the helix-loop-helix motif has been replaced with a random 9 amino acid sequence. Most preferably still, the FASTbodies according to the invention comprises, preferably consists of an adipocyte fatty acid binding protein (a-FABP) in which the helix-loop-helix motif is replaced by a random amino acid sequence, preferably the helix-loop-helix motif is replaced by a random 9 amino acid sequence.

The present inventors consider that the scaffold structure of the invention may be compared to an antibody structure, where the individual loop regions compare to the different CDR regions of the antibody. Specifically, the inventors consider that in the case where the FASTbodies is based on a fatty acid binding protein in which the helix-loop-helix motif is replaced with a peptide, preferably a 9 amino acid random amino acid sequence, then the peptide sequence may be compared to Heavy Chain CDR3 regions, since this position in the scaffold can accommodate significant variations in length and structure without disturbing the overall fold of the scaffold.

The essential features of a scaffold according to the invention are based on the results of these experiments. In particular, the present inventors have found that the β-strand clam structure appears to be important for determining the structural integrity of the ligand binding scaffold of the invention, whilst the loop regions appear to determine the ligand binding specificity. Moreover, the inventors consider that the absence of disulphide bridge forming cysteines in the fatty acid binding scaffold (FASTbodies) according to the invention is necessary to permit the correct folding of the FASTbodies within an intracellular environment. Thus, the inventors consider that the latter feature is essential for specific ligand binding to a FASTbodies according to the invention within an intracellular environment.

In a further aspect, the present invention provides a method for generating a fatty acid binding protein scaffold (FASTbody) which method comprises:
(a) Providing a single polypeptide chain fatty acid binding protein, or the nucleic acid encoding it; and
(b) Randomizing the helix-loop-helix motif, or the nucleic acid encoding it.

According to the above aspect of the invention, the term 'randomizing' (the helix-loop-helix motif) of the FASTbody of the invention refers to the process of altering the amino acid sequence of the motif such that the resultant amino acid sequence of the 'randomized' helix-loop-helix motif is not 100% identical to the helix-loop-helix motif comprised by the fatty acid binding protein from which the FASTbody of the invention is generated.

'Randomization of an amino acid sequence' as herein defined may be achieved using any suitable technique which results in changing at least one amino acid of the relevant amino acid sequence so that the resultant amino acid sequence is not 100% identical to the same amino acid sequence prior to the 'randomization process'. Suitable techniques include mutation, deletion, insertion, translocation of nucleic acids encoding some or all of the relevant region. Alternatively, randomization as herein defined may be achieved at the amino acid level using similar techniques, namely, mutation, deletion, insertion, translocation of one or amino acids comprising the relevant sequence. In a preferred embodiment of the above aspect of the invention 'randomization' is achieved by replacing part or all of the relevant region (for example part or all of the helix-loop-helix motif) by a second amino acid sequence. Such replacement may be achieved either at the nucleic acid level or at the amino acid level. This method is described in more detail in the Examples.

Those skilled in the art will appreciate that a FASTbody according to the invention may be generated at the protein/polypeptide level or at the nucleic acid level. Thus in the case that the FASTbody is generated at the polypeptide level, then the helix-loop helix motif of a fatty acid binding protein/polypeptide is randomized. In contrast, in the case that the FASTbody according to the invention is prepared at the nucleic acid level then, the nucleic acid encoding a helix-loop-helix motif of a fatty acid binding protein is 'randomized', as defined herein.

According to the above aspect of the invention, advantageously, the method of the invention comprises a further step (c) in which one or more loop regions connecting β-strands designated A, B, C, D, E, F, G, H, I and/or J, or the nucleic acid encoding them is randomized, as herein defined. According to this preferred embodiment of the above aspect of the invention, preferably, the loop region connecting β-strands B and E is randomized as defined herein. More advantageously still, the loop regions connecting β-E and β-F, β-G and β-H and β-I and β-J are randomized as herein defined.

According to the above aspect of the invention, preferably, the fatty acid binding protein is adipocyte fatty acid binding protein (FABP-A). Sources of suitable fatty acid binding proteins will be familiar to those skilled in the art and are described in the detailed description of the invention.

According to the above aspect of the invention, the term 'replacing' (the helix-loop-helix motif) refers to the process of removing the helix-loop-helix motif and inserting in its place a suitable peptide sequence.

'Suitable' peptide sequences for use according to the method of the invention may be of any length from 3 amino acids to 100 amino acids. Advantageously, a suitable peptide sequence is from 3 to 50, or 3 to 20 amino acids in length, more preferably, it is from 3 to 15 amino acids in length. More preferably still, it is from 3 to 10 amino acids in length. Most preferably, where the peptide is one replacing the helix-loop-helix motif, then the peptide sequence according to the invention is preferably 9 amino acids in length. Moreover, in the case where the peptide functions to replace the loop connecting β-strands B and E, then the peptide is preferably 5 amino acids long or 7 amino acids long. Advantageously, a peptide for use according to the method of the invention is a 'random peptide' sequence as herein described. Those skilled in the art will appreciate that the peptide according to step (b) above may or may not comprise secondary structural elements.

According to the present invention, the term 'random peptide' refers to an amino acid sequence of no defined sequence. That is it refers to an amino acid sequence of any sequence. Such a random peptide may be generated using any method known to those skilled in the art. Advantageously, a 'random' peptide according to the present invention will be generated using a peptide synthesiser.

In a further aspect still, the present invention provides a fatty acid binding protein scaffold (FASTbody) obtainable according to the method of the invention.

Advantageously, the FASTbodies according to the invention comprises, preferably consists of one of the fatty acid binding proteins selected from the group consisting of : A-FABP, I-FABP, L-FABP, H-FABP, E-FABP, IL-FABP, B-FABP, M-FABP and T-FABP in which the helix-loop-helix motif is 'randomized' as herein defined. Preferably the helix-loop-helix motif is replaced with a 9 amino acid random peptide sequence described herein.

More advantageously, the FASTbody according to the invention comprises, preferably consists of one of the fatty acid binding proteins selected from the group consisting of: A-FABP, I-FABP, L-FABP, H-FABP, E-FABP, IL-FABP, B-FABP, M-FABP and T-FABP in which the helix-loop-helix motif has been randomized as herein defined and additionally one or more of the loop regions connecting one or more of the β-strands designated A, B, C, D, E, F, G. H. I and J is 'randomized' as herein described.

More advantageously, a FASTbody according to the invention comprises, preferably consists of a-FABP in which the helix-loop-helix region has been replaced with a randomized peptide sequence, preferably the 9 amino acid and additionally the loop regions connecting one or more of the β-strands designated A, B, C, D, E, F, G. H. I and J is 'randomized' as herein described

Most advantageously, a FASTbodies according to the invention comprises, preferably consists of a-FABP in which the helix-loop-helix region has been replaced with a random peptide sequence as defined herein, preferably the 9 amino acid random peptide described in the Examples and additionally the loop regions connecting the β-strands designated E and F is 'randomized' as herein described. In a further preferred embodiment of the above aspect of the invention, a FASTbody according to the invention comprises, preferably consists of a-FABP in which the helix-loop-helix region has been replaced with a random peptide sequence as defined herein, preferably the 9 amino acid random peptide described in the Examples and additionally the loop regions connecting the β-strands designated E and F, G and H and I and J are 'randomized' as herein described.

In a further aspect the present invention provides a nucleic acid construct encoding a fatty acid binding protein scaffold (FASTbody) according to the invention.

In a further aspect still, the present invention provides a vector comprising a nucleic acid construct according to the invention.

In a further aspect still, the present invention provides a host cell comprising a vector according to the invention.

Thus in a further aspect the present invention provides a method for changing the ligand binding specificity of a fatty acid binding scaffold (FASTbody) according to the invention, which method comprises the steps of:
(a) Providing a fatty acid binding scaffold according to the invention, and
(b) Randomizing one or more loops connecting the β-stands designated A,B, C, D, E, F, G, H, I and J.

In a further aspect still the present invention provides a library of fatty acid binding protein scaffolds (FASTbodies) according to the invention.

According to the above aspect of the invention, advantageously, the FASTbodies according to the invention comprises, preferably consists of one of the fatty acid binding proteins selected from the group consisting of : A-FABP, I-FABP, L-FABP, H-FABP, E-FABP, IL-FABP, B-FABP, M-FABP and T-FABP in which the helix-loop-helix motif is 'randomized' as herein defined. Preferably the helix-loop-helix motif is replaced with a 9 amino acid random peptide sequence described herein.

More advantageously, the FASTbodies according to the invention comprises, preferably consists of one of the fatty acid binding proteins selected from the group consisting of : A-FABP, I-FABP, L-FABP, H-FABP, E-FABP, IL-FABP, B-FABP, M-FABP and T-FABP in which the helix-loop-helix motif has been replaced with a randomized amino acid sequence, preferably a 9 amino acid random peptide sequence described herein and additionally one or more of the loop regions connecting one or more of the β-strands designated A, B, C, D, E, F, G. H. I and J is 'randomized' as herein described.

More advantageously, a FASTbody according to the invention comprises, preferably consists of a-FABP in which the helix-loop-helix region has been replaced with a randomized peptide sequence, preferably the 9 amino acid and additionally the loop regions connecting one or more of the β-strands designated A, B, C, D, E, F, G. H. I and J is 'randomized' as herein described

Most advantageously, a FASTbody according to the invention comprises, preferably consists of a-FABP in which the helix-loop-helix region has been replaced with a random peptide sequence as defined herein, preferably the 9 amino acid random peptide described in the Examples and additionally the loop regions connecting the β-strands designated E and F is 'randomized' as herein described. In a further preferred embodiment of the above aspect of the invention, a FASTbody according to the invention comprises, preferably consists of a-FABP in which the helix-loop-helix region has been replaced with a random peptide sequence as defined herein, preferably the 9 amino acid random peptide described in the Examples and additionally the loop regions connecting the β-strands designated E and F, G and H and I and J are 'randomized' as herein described.

In a further aspect still the present invention provides a method for selecting a fatty acid binding scaffold according to the invention which scaffold is capable of binding to a defined ligand which method comprises the steps of:
(a) Providing a fatty acid binding protein (FASTbody) library according to the invention,
(b) Testing the ability of the library according to step (a) to bind the defined ligand; and
(c) Selecting those fatty acid binding scaffolds according to step (b) which are capable of binding to the defined ligand.

The present inventors have surprisingly found that the binding of specific ligand to the FASTbodies according to the invention is modulated by the presence or absence of fatty acid binding to the FASTbody.

Thus in a further aspect the present invention provides the use of a fatty acid in modulating the binding of specific ligand to a FASTbodies according to the invention.

In a further aspect still, the present invention provides the use of a fatty in monitoring the binding of FASTbodies to one or more specific ligands.

According to the aspect of the invention referred to above, advantageously the use comprises measuring the amount of fatty acids bound to the FASTbody. Upon ligand binding to FASTbody the dissociation rate of fatty acid from FASTbody will be altered, thus allowing a correlation to be obtained.

As used herein the term 'fatty acid' includes within its scope fatty acid derivatives, homologues, analogues and/or fragments thereof so long as such derivatives, homologues, analogues and/or fragments thereof possess the requisite activity of FASTbody binding and the consequent modulation of specific ligand binding to a FASTbody as herein described.

According to the above aspect of the invention, preferably the fatty acid is one or more selected from the groupconsisting of the following: Lauric acid, Myristic acid, Myristoleic acid, Palmitic acid, Palmitoleic acid, Steric acid, Oleic acid, Linoleic acid, Linolenic acid, Arachidic acid, Arachidonic acid, Docosahexaenoic acid or fluorescent analogues of the above. Those skilled in the art will appreciate that this list is not intended to be exhaustive.

The present inventors consider that FASTbodies and/or libraries thereof according to the invention may be used to generate validation systems suitable for use in combination with high throughput screens of new chemical entities.

Thus in a final aspect the present invention provides a method of identifying a potential drug candidate which is capable of displacing the binding of a FASTbody according to the invention to the target antigen, which method comprises the steps of:
(a) Providing a library of FASTbodies according to the invention,
(b) Providing a sample of one or more drug candidates,
(c) Screening the library for the ability of the one or more drug candidates to displace the binding of one or more FASTbodies within the library according to step (a).

According to the above aspect of the invention, those molecules which are found to displace the binding of one or more FASTbodies in a library according to the invention represent potential drugs candidates. Preferably the method according to the above aspect of the invention may be effected by using high throughput screening technology. Suitable technology will be familiar to those skilled in the art.

### DEFINITIONS.

The term 'library' of FASTbodies according to the present invention refers to a mixture of heterogeneous polypeptides or nucleic acids. The library is composed of members, which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form organisms or cells, for example bacteria, viruses, animal or plant cells and the like, transformed with a library of nucleic acids. Preferably, each individual organism or cell contains only one member of the library. Advantageously, the nucleic acids are incorporated into expression vectors, in order to allow expression of the polypeptides encoded by the nucleic acids.

**'Randomization of an amino acid sequence'** as herein defined may be achieved using any suitable technique which results in changing at least one amino acid of a relevant amino acid sequence so that the resultant amino acid sequence is not 100% identical to that amino acid sequence prior to the 'randomization process'. Suitable techniques include mutation, deletion, insertion, translocation of nucleic acids encoding some or all of the relevant region. Alternatively, randomization as herein defined may be achieved at the amino acid level using similar techniques, namely, mutation, deletion, insertion, translocation of one or amino acids comprising the relevant sequence. In a preferred embodiment of the above aspect of the invention 'randomization' is achieved by replacing part or all of the relevant region (for example part or all of the helix-loop-helix motif of a FASTbody according to the invention) by a second amino acid sequence. Such replacement may be achieved either at the nucleic acid level or at the amino acid level.

**"Antibodies"** are defined herein as constructions using the binding (variable) region of such antibodies, and other antibody modifications. Thus, an antibody useful in the invention may comprise whole antibodies, antibody fragments, polyfunctional antibody aggregates, or in general any substance comprising one or more specific binding sites from an antibody. The antibody fragments may be fragments such as Fv, Fab and F(ab')₂ fragments or any derivatives thereof, such as a single chain Fv fragments. The antibodies or antibody fragments may be non-recombinant, recombinant or humanized. The antibody may be of any immunoglobulin isotype, e.g., IgG, IgM, and so forth. In addition, aggregates, polymers, derivatives and conjugates of immunoglobulins or their fragments can be used where appropriate.

According to the present invention, the term **'random peptide'** refers to an amino acid sequence of no defined sequence. That is it refers to an amino acid sequence of any sequence. Such a random peptide may be generated using any method known to those skilled in the art. Advantageously, a 'random' peptide according to the present invention will be generated using a peptide synthesiser.

**CDR (complementarity determining region)** of an immunoglobulin molecule heavy and light chain variable domain describes those amino acid residues which are not framework region residues and which are contained within the hypervariable loops of the variable regions. These hypervariable loops are directly involved with the interaction of the immunoglobulin with the ligand. Residues within these loops tend to show less degree of conservation than those in the framework region.

**Intracellular** means inside a cell. The cell may be any cell, prokaryotic or eukaryotic, and is preferably selected from the group consisting of a bacterial cell, a yeast cell and a higher eukaryote cell. Most preferred are yeast cells and mammalian cells. In addition the term 'Intracellular' refers to environments which resemble or mimic an intracellular environment. Thus, "intracellular" may refer to an environment which is not within the cell, but is *in vitro.*

**Specific binding** in the context of the present invention, means that the interaction between the FASTbody and the ligand are specific, that is, in the event that a number of molecules are presented to the FASTbody, the latter will only bind to one or a few of those molecules presented. Advantageously, the FASTbody-ligand interaction will be of high affinity. The interaction between FASTbody and ligand will be mediated by non-covalent interactions such as hydrogen bonding and Van der Waals. Generally, the interaction will occur on the loop regions of the FASTbody.

### BRIEF DESCRIPTION OF THE FIGURES.

**Figure 1** shows a schematic representation of the adipocyte fatty acid binding protein. The 10 β-strands named A, B, C, D, E, F, G, H, I, J are represented at squares, while the Helix-loop-Helix motif connecting β-strand A and B are shown as cylinders. The loops connecting the secondary structural elements are represented a thin lines.
**Figure 2** shows a 3-dimensional representation of the structure of Adipocyte Fatty Binding Protein Complexed With 1-Anilino-8-Naphthalene Sulfonate created using the program Swiss-PDB viewer (http://www.expasy.org/spdbv/). The coordinates (2ANS) were taken from PDB.
**Figure 3** show the sequence comparison between human and mouse Adipocyte Fatty Acid Binding protein, with the secondary structural elements shown. Helix regions are shown as squares and b-strands are shown as lines ending with an arrow, loop regions are indicated with the letter t.
**Figure 4** shows the transcribed gene sequence of wild type human adipocyte fatty acid binding protein, derived from genebank with accession number : NM 001442. Highlighted in yellow is the translated sequence.

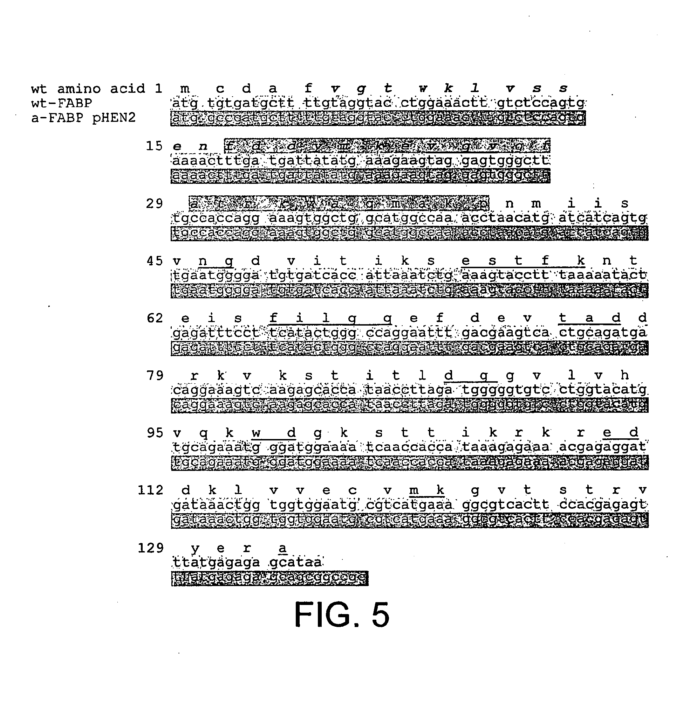

**Figure 5** shows a sequence comparison between wild type human adipocyte fatty acid binding protein and the modified recombinant adipocyte fatty acid binding protein cloned in the phagemid vector pHEN2. In the first line the wild type amino acid sequence of A-FABP is given with secondary structural elements (italics indicate helix, blue indicate sheet and black underscored loop residues).

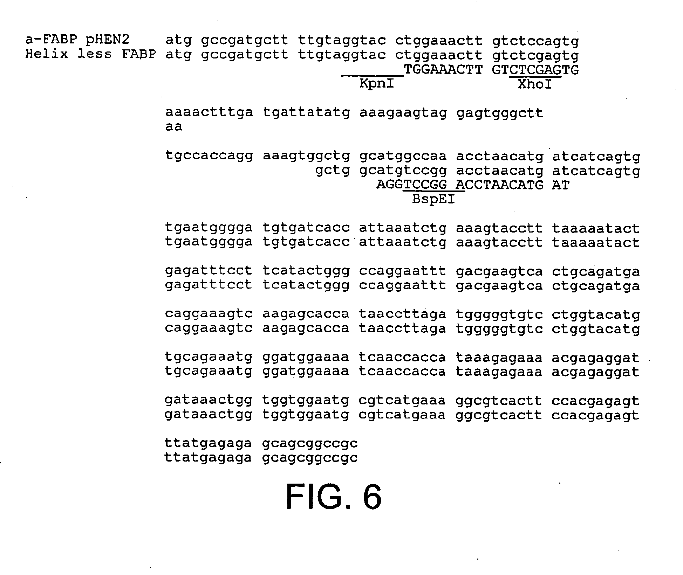

**Figure 6** shows the sequence comparison between the modified recombinant adipocyte Fatty acid Binding protein in pHEN2 before and after removal of the helix loop helix region. In the Helix less variant of the adipocyte fatty acid binding protein the unique restriction enzyme recognition sites for KpnI, XhoI and BspE1 were added as detailed in Example 1.

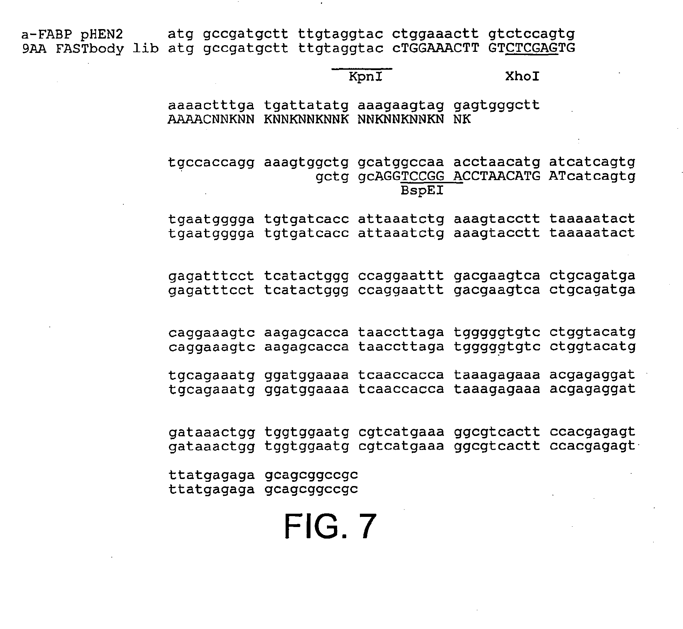

**Figure 7** shows the sequence comparison between the Helix less variant of the adipocyte fatty acid binding protein and the first FASTbody library in which the helix loop helix region of the adipocyte fatty acid binding protein was replaced by a randomized 9 amino acid insert. In the randomization process the codon NNK is used.

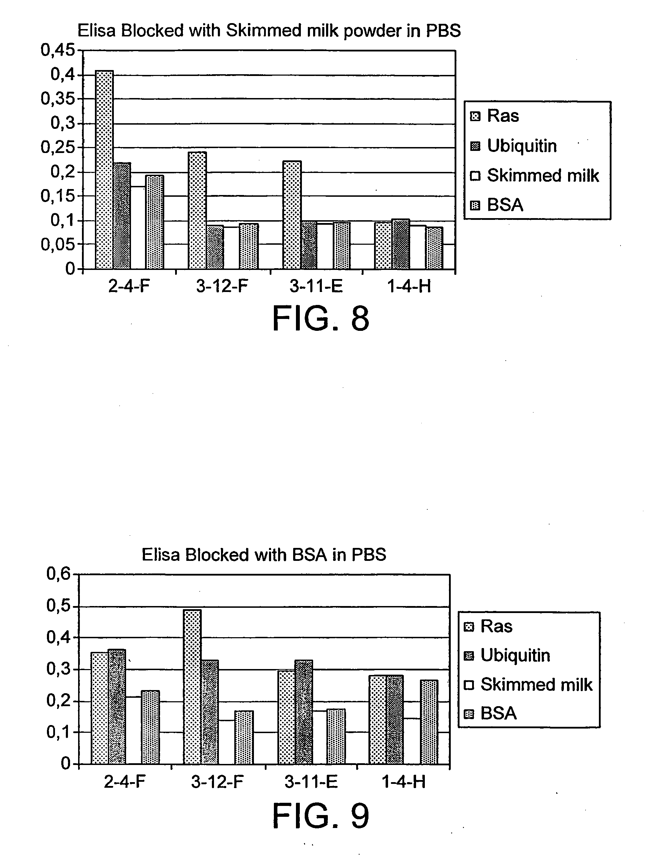

**Figure 8** shows the ELISA results obtained with 4 selected FASTbody binders selected against RAS. In the ELISA the wells were coated with 50 ng of each of the indicated antigens (RAS, Ubiqutin, Skimmed milk and BSA). To prevent binding to free plastic surface the wells were bloked by adding 3 % skimmed milk powder in PBS, followed by incubation with 50 µl of a phage supernatant of the individual FASTbody binders (2-4-F, 3-12-F, 3-11-E and 1-4-H). FASTbody binding were detected by reacting with a monoclonal antibody against the phage particle followed by development with OPD. The three binders 2-4-F, 3-12-F and 3-11-E binds RAS specifically while 1-4-H does not show significant binding.
**Figure 9** shows the ELISA results obtained with the same 4 selected FASTbody binders as shown in figure 8. In the ELISA the wells were coated with 50 ng of each of the indicated antigens (RAS, Ubiqutin, Skimmed milk and BSA). To prevent binding to free plastic surface the wells were bloked by adding 2% BSA in PBS, followed by incubation with 50 µl of a phage supernatant of the individual FASTbody binders (2-4-F, 3-12-F, 3-11-E and 1-4-H). BSA is known to bind Fatty acids strongly, therefore any fatty acid bound to the FASTbody structures would be displaced by blocking and incubating the FASTbodies in the presence of BSA. FASTbody binding were detected by reacting with a monoclonal antibody against the phage particle followed by development with OPD. FASTbodies from clone 2-4-F and 3-11-E does not show a significant binding to RAS compared to the background, thus indicating that the RAS recognition of these FASTbodies are depended on fatty acids being present in the FASTbody scaffold. 3-12-F retain some of the binding specificity toward RAS.

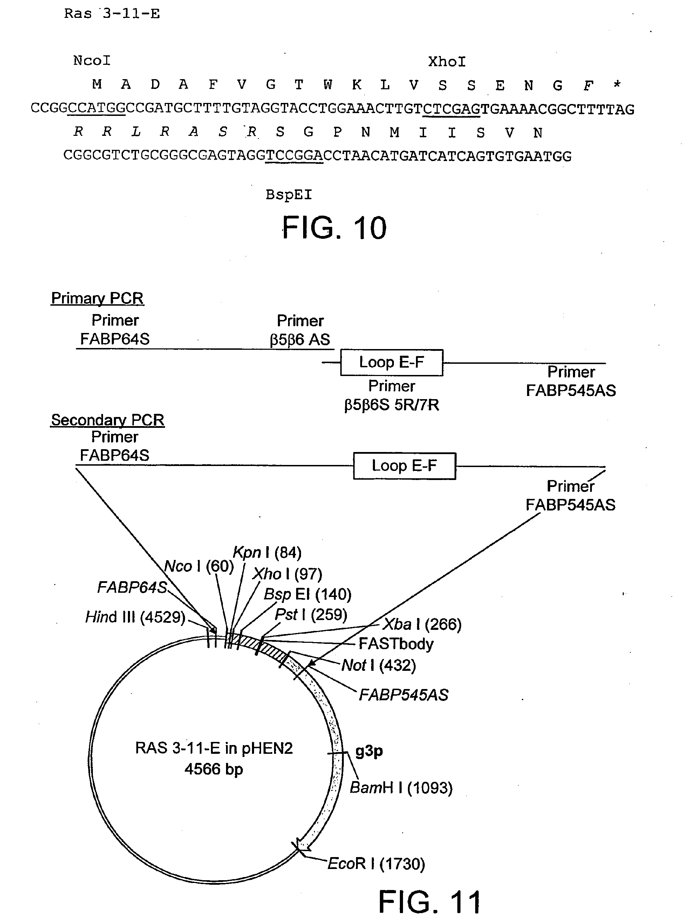

**Figure 10** show part of the sequence of the binder 3-11-E selected for binding against the RAS protein. Only part of the sequence surrounding the 9 amino acid sequence is shown. The restriction site NcoI is located at the start codon for the FASTbodies, while XhoI and BspE1 is located at either site of the 9 amino acid insert. The 9 amino acids are shown in italics.
**Figure 11** shows a schematic representation of the library constructions based on the binder 3-11-E. As outlined in Example 3. Two PCR reactions were performed using the binder 3-11-E in pHEN2 as template. One using the an oligo priming in the vector sequence upstream of the start codon of the FASTbody (FABP64S) together with an oligo immediately in front of the loop connecting b-strand E and F, and the other using an oligo priming in the vector sequence downstream of the end of the FASTbody (FABP545AS) together with two different oligos used to insert a random sequence of 5 and 7 amino acids respectively in the loop connecting b-strand E and F.
   A secondary PCR amplification was used to assemble the two fragments before cloning the PCR products in the Pst1 and Not1 sites.

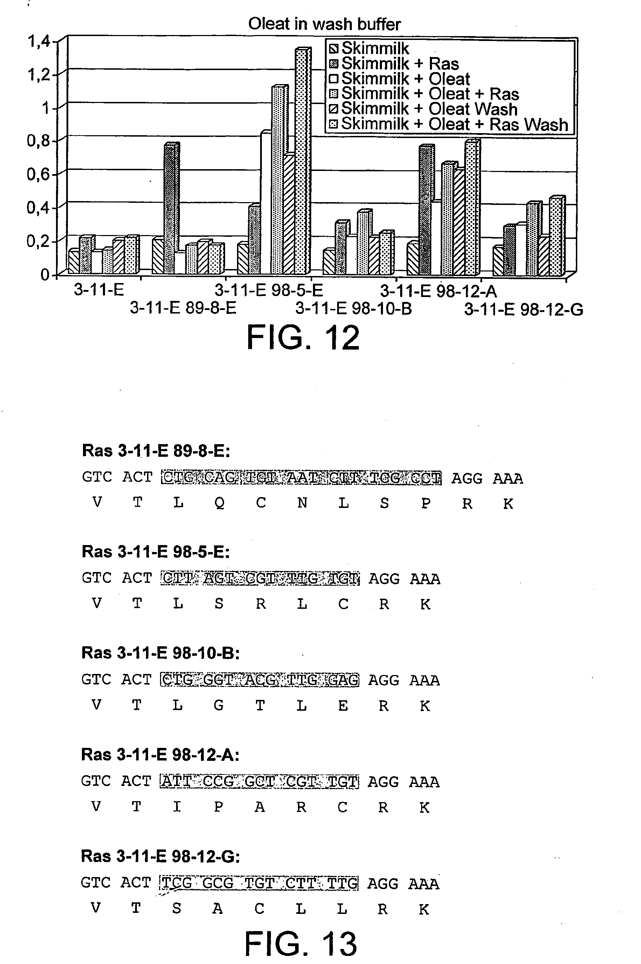

**Figure 12** show the ELISA results of a selected set of binders obtained from the two FASTbody libraries constructed using the binder 3-11-E previously selected against RAS as template. The two libraries were randomised as previously shown in Example 3.
   Five of the positive clones obtained after selection of the FASTbody libraries generated using 3-11-E as template and the binder 3-11-E were tested in an ELISA designed to underline the importance of adding free fatty acids to the FASTbodies. The binders were allowed to bind to skimmed milk powder alone, Ras blocked with skimmed milk powder,
   Skimmed milk powder an oleate, RAS with oleate and blocked with skimmed milk poweder, Skimmed milk poweder with Oleate and Oleate present at all steps of washing and incubation, Ras with Oleate and blocked with skimmed milk powder and Oleate present at all steps of washing and incubation.
   While all of the clones from the new libraries generated binders with increased binding to RAS, the influence of Oleate present only in the coating step or in all other steps of washing and incubating varied between the clones.
**Figure 13** shows the sequences of the 5 binders selected from the FASTbody libraries based on the 3-11-E binder with randomisation of the loop connecting b-strand E and F.
   The randomised residues are marked as gray on the DNA level. All the clones have different sequences establishing the potential of generating a divers array of binders using the FASTbody scaffold. Furthermore binders with both 5 and 7 randomised amino acids was identified establishing the flexibility of the loop regions to accommodate loop sequence of varying length.

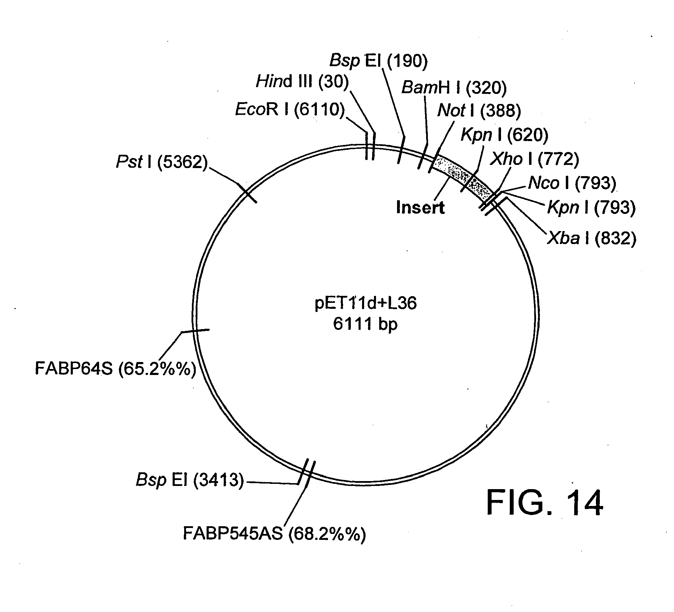

**Figure 14** show a modified pET11d vector where cloning into the NcoI and NotI allow expression of a fusion protein with a myc and His tag added at the C-Terminal end of the fusion protein.
**Figure 15** shows a schematic representation of the expression vector allowing intracellular expression of the FASTbodies in Mucor.
**Figure 16** shows the detailed sequence of the vector pEUKA7-kan.
**Figure 17** show the intracellular expression of the RAS binder 3-11-E 89-8-E expressed from the vector pEUKA7-kan. As a control the KFA143 (Appel et al. 2004) were grown as under aerobic and anaerobic conditions.
**Figure 18** schematically show the assembly of the synthetic gene fragment for creation of the total randomized FASTbody library as described in example 7.
**Figure 19** shows an ELISA experiment on FASTbodies a total randomised library. The ELISA were performed as described in example 8 and the results are given as absorbance at 490 nm. Postive clones are marked with grey shading.

### DETAILED DESCRIPTION OF THE INVENTION.

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference) and chemical methods.

### Fatty acid binding proteins.

Fatty acid binding scaffolds (FASTbodies) according to the present invention may be generated from one or more fatty acid binding proteins.

Such fatty acid binding proteins may be categorised as shown in **Table 1.**

| Tissue | FABP name | FABP abbrevation |
|---|---|---|
| Liver, intestine, kidney, stomach | Liver | L |
| Intestine, stomach | Intestinal | I |
| Heart, kidney, skeletal muscle, aorta, adrenal, placenta, brain, testes, ovary, lung, mammary gland, stomach | Heart | H |
| Adipose tissue | Adipocyte | A |
| Skin, brain, lens, capillary, endothelium, retina | Epidermal | E |
| Intestine, ovary, adrenals, | Ileal | IL |
| stomach | | |
| Brain | Brain | B |
| Peripheral nervous system | Myelin | M |
| Testis | Testicular | T |

In a preferred embodiment of the invention, FASTbodies according to the invention is generated from A-FABP (adipocyte fatty acid binding protein).

### Structural features of fatty acid binding proteins.

Fatty acid binding proteins from which a FASTbody according to the present invention may be generated comprises a large β-strand region which forms a β-clam structure as defined herein and a small a helical region. Together these structures (the characteristics of which are described below) create a specific fatty acid binding pocket.

The first reported crystallographic studies to enter the literature were of recombinant rat I-FABP. Structural analyses of several FABPs have revealed markedly similar three-dimensional folds consisting of 10 antiparallel β-strands that form a β-barrel (Zanotti G. 1999). This β-barrel is capped by two short α-helices arranged in a helix-loop-helix structure (figure 1). The present evidence suggests that the helix-loop-helix structure together with the turns connecting β-strands C-D and D-E, functions as a "dynamic portal" that regulates Fatty Acid entry and exit from the internal ligand binding cavity. In particular the transfer of Fatty Acids to membranes seems to be controlled by the helix-loop-helix motif (Liou H-L et al 2002, Córsico B 2004)). This class of FABP's, to which H-FABP also belongs, has been termed membrane-active FABPs. These catalyses both the dissociation of the fatty acid from the donor membrane and binding to the acceptor membrane (Glatz J.F.C. 2003).

The topology of the FABP's is comparable to two other families of closely related structural families, namely the Lipocalins and the streptavidins. However whereas the structure of the FABP family comprises a 10 β-sheet clam structure with a helix-loop-helix lid, the two others comprise structures of 8 β-stand and no helix-loop-helix motif. The FABP barrel is more flattened and elliptical than either that of lipocalins or streptavidin. Based on the structural similarities the three distinct families has been suggested to form part of a larger group, the calycin structural superfamily (Flower D.R. 1993). In contrast to the remarkably similar structures, the members of the FABP family show an amino acid sequence similarity of 22-73 %, with 39 highly conserved residues.

Some details of these structural topologies are presented below:

### (a) β-Strands.

In the beta (β) strand structure, the polypeptide segment adops an extended conformation, where the phi- and psi-angles are around -120° and +120° respectively. The R groups of the amino acids alternates in location with respect to the plane defined by the backbone in such a way that every second is located on one side of the plan and every other second on the opposite side of the plan.

### (b) α-helix.

In the alpha helical structure, the polypeptide backbone is arranged in a helical coil having about 3.6 residues per turn. The R groups of the amino acid extend outwards from the tight helix formed from the backbone. In such a structure the repeat unit consisting of a single complete turn of the helix, extends about 0.54 nm along the long-axis. The alpha helix is the simplest arrangement which can be adopted by a polypeptide taking into account the constraint imposed by the planar peptide bonds.

### (c) β-Clam structure.

The β-clam topology consists of two-five stranded anti-paralell β-sheets surrounding a large solvent-filled cavity within which the ligand binds. This β-clam topology is present in all members of the fatty acid binding protein family and is an essential feature of the fatty acid binding protein scaffold (FASTbodies) according to the invention.

### (a) Helix-loop helix motif.

Fatty acid binding proteins comprise two α-helices joining beta-strands A and B. That is β-strands A and B are joined by a helix-loop helix motif. The fatty acid binding protein scaffold (FASTbodies) according to the invention retains this feature in certain embodiments. The present inventors consider that the presence of the helix-loop helix motif is not required in order to maintain the integrity of the fatty acid binding cavity but may serve to regulate the affinity of fatty acid binding (Cistola, D. P 1996).

### FATTY ACID BINDING SCAFFOLDS ACCORDING TO THE INVENTION.

In a first aspect the present invention provides a fatty acid binding protein scaffold (FASTbody) capable of specific binding to one or more ligands, which scaffold comprises a single-chain polypeptide with the following structural properties:
(a) The scaffold contains 10 β-strands (designated ABCDEFGHI and J) connected by loop regions which determine the specificity of ligand binding, wherein the β-stands together form a β-clam structure; and wherein
(b) The loop regions connecting β-A and β -B; β -C and -D; β -E and -F; β -G and β -H; β-I and β-J are located on the same site of the β-clam structure; wherein the fatty acid binding scaffold does not contain any disulphide bridge forming cysteines; wherein the scaffold does not comprise a helix-loop-helix motif.

### Characteristics of fatty acid binding protein scaffolds according to the invention.

The present inventors have studied structure/function relationships in members of the fatty acid binding protein family. They have used the information obtained to design a scaffold. Importantly, such a scaffold consists of loop regions which are capable specific interaction with one or more ligands. Further they have used this information to examine the influence of fatty acids associated with the scaffold structure and the influence of said fatty acids on binding of ligands. Some of the structural considerations taken into account in designing a fatty acid binding scaffold (FASTbody) scaffold according to the present invention are listed below:

### (a) Structural flexibility of turns:

When the structure of the fatty-acid-binding protein from the parasitic platyhelminth Echinococcus granulosus (Eg-FABP1) is compared to another structural family member the P2 myelin protein, the major structural differences occur in the turns before and after β-stand H (Jakobsson E. 2003). Despite structural differences in the turn regions surrounding β-strand H, the conformation and location of this strand are identical in the two protein, thus suggesting that a certain degree of sequence variation is allowed in turn regions, while still preserving the overall structural features.

Previously studies have investigated the influence of mutating single amino acids in I-FABP. The residues were chosen on the basis of a likely influence on folding or stability. Leu64 located in the loop connecting β-strand D and β-stand E makes numerous contacts with residues in other stands, and mutants at residue 64 exhibited a marked decrease in stability (Rajabzadeh M. 2003). While this may be true for certain conserved key residues, a large number of mutants of other FABP proteins have been examined an exhibit similar or moderately decreased stabilities with a conservation of the overall structural characteristics (Zimmerman A.W., et al. 1999)

The high flexibility which is needed for proper uptake and delivery of fatty Acids, mostly involves flexibility of the helix-loop-helix region, while the overall conformational stability of the β-clam structure is preserved, as has been shown for the I-FABP and L-FABP (Constantine K.L. et al. 1998; Arighi C.N. 2003; Córsico B. 2004).

### (b) Stability of fatty acid binding proteins (FABPs)

The overall FABP structure exhibit significant conformational stability, although a significant spread in stabilities exists between the individual members. Stability measured by Urea denaturation show that H-FABP is the most stable with a midpoint of denaturation at 5.95 M Urea, this is followed by A-FABP (5.36 M), I-FABP (5.20 M), B-FABP (4.07 M), Il-FABP (3.78 M), M-FABP (3.00 M), E-FABP (2.57 M) and L-FABP (1.85 M) (Zimmerman A.W. 2001). This conformational stability does not correlate with the affinity for ligand binding.

### Helix-less I-FABP:

Previous studies produced a helix-less variant of the rat I-FABP in order to examine the role of the helix-loop-helix motif in ligand binding (Wu F., et al. 2001). In the helix-less variant the helix-loop-helix motif was replaced by a ser-gly linker. Circular dichroism and NMR spectra indicated that this I-FABP variant has a high β-sheet content and a β-clam topology similar to that of the wild-type protein (Steele R.A. et al. 1998). The backbone conformation of the helix-less variant is nearly superimposable with the β-sheet domain of wild-type I-FABP. The stability of the helix-less variant is slightly reduced upon denaturation with guanidine treatment. Ligand associations rates for the helix-less variant and the wild-type protein were comparable, but the dissociation rates was 16-fold lower for the wild-type protein. The present inventor has shown that upon binding of a ligand to the FASTbodies of the present invention, the dissociation rate of fatty acids binding to the FASTbodies will be altered, thus allowing a correlation between ligand binding and dissociation rate of fatty acids to be made. These data indicate that the α-helix of I-FABP are not required to maintain the integrity of the fatty acid binding cavity but may serve to regulate the affinity of fatty acid binding (Cistola D.P. 1996). Furthermore fatty acid transfer studies showed that in the absence of the α-helical domain, effective collisional transfer of fatty acids to phospholipids membranes does not occur, indicating that the α-helical region of FABP is essential for interaction with membranes.

For a number of single point mutations as well as chimeric H-FABP and A-FABP conservation of the overall secondary structure has been observed (Richier G.V. et al 1998, Liou H-L 2002) further strengthening the notion that sequence variation can be accommodated without destroying the overall fold of the FABP.

### FASTbody LIBRARIES ACCORDING TO THE PRESENT INVENTION.

The present inventors used the information obtained by studying the structure/function relationships of fatty acid binding proteins described above in order to design a FABP scaffold according to the invention. Libraries of such molecules were then created by the present inventors in order to select FASTbodies according to the present invention which exhibit one or more desired ligand binding specificities. Preferably the one or more ligands is RAS.

An example of one strategy used in the generation of a specific-ligand FASTbody library according to the present invention is provided below:

First the helix-loop-helix region, was replaced by a random 9 amino acid peptide. The library was selected on RAS and binders obtained. In the next step one of the binders isolated by selecting on RAS was chosen as for randomization of the loop connecting β-strand E and F, as herein defined. Two different libraries were created, one having 5 randomised amino acids replacing the loop and one having 7 randomised amino acids replacing the loop. The new libraries were again selected for binding toward RAS. A number of clones were isolated with increased binding affinity as judge from ELISA experiments.

An unforeseen result came when the blocking reagent was changed from the normally used 2 % skimmed milk powder in PBS to 2 % BSA in PBS. The change in blocking reagent resulted in decreased signal for some of the isolated binders toward RAS. BSA binds and transports Fatty Acids in the serum, consequently BSA has a significant affinity for Fatty Acids. In previous studies it has been shown that the binding affinity of a helix-less variant of I-FABP has a 20-100 reduced binding affinity toward Fatty Acids.

Given that a similar reduction of the binding affinity for Fatty Acids exist for the present engineered scaffold build on the A-FABP, the above results indicated that the presence of Fatty Acids in the selected scaffold may regulate the binding of individual scaffold to their targets.

Based on this novel observation regulatable binders toward predefined target potentially can be isolated. Further a dependence on fatty acids present in the FASTbody can be used to measure ligand binding to the FASTbody by measuring the fatty acid binding of the FASTbody.

Following the proof of concept described above a new library has been constructed in which the helix-loop-helix region is replaced with the random 9 amino acid peptide, described previously, together with randomization of the loop regions connecting β-E and β -F; β -G and β -H; β -I and β -J.

The resulting library has been selected against various targets, including RAS, and a diverse series of binders has been obtained.

### Preparation of FASTbody libraries according to the invention.

The term 'library' of FASTbodies according to the present invention refers to a mixture of heterogeneous polypeptides or nucleic acids. The library is composed of members, which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form organisms or cells, for example bacteria, viruses, animal or plant cells and the like, transformed with a library of nucleic acids. Preferably, each individual organism or cell contains only one member of the library. Advantageously, the nucleic acids are incorporated into expression vectors, in order to allow expression of the polypeptides encoded by the nucleic acids.

Libraries of (fatty acid binding protein scaffolds) FASTbodies according to the present invention may be prepared using any suitable method known to those skilled in the art.

### Library vector systems

A variety of selection systems are known in the art which are suitable for use in the present invention. Examples of such systems are described below.

Bacteriophage lambda expression systems may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87*;* Mullinax et al. (1990) Proc. Natl. Acad. Sci. U.S.A., 87: 8095; Persson et al. (1991) Proc. Natl. Acad. Sci. U.S.A., 88: 2432) and are of use in the invention. Whilst such expression systems can be used to screening up to 10⁶ different members of a library, they are not really suited to screening of larger numbers (greater than 10⁶ members).

Of particular use in the construction of libraries are selection display systems, which enable a nucleic acid to be linked to the polypeptide it expresses. As used herein, a selection display system is a system that permits the selection, by suitable display means, of the individual members of the library by binding the generic and/or target ligands.

### Phage display libraries.

Selection protocols for isolating desired members of large libraries are known in the art, as typified by phage display techniques. Such systems, in which diverse peptide sequences are displayed on the surface of filamentous bacteriophage (Scott and Smith (1990) Science, 249: 386), have proven useful for creating libraries of antibody fragments (and the nucleotide sequences that encoding them) for the *in vitro* selection and amplification of specific antibody fragments that bind a target antigen (McCafferty *et al.,* WO 92/01047). The nucleotide sequences encoding the V_{H} and V_{L} regions are linked to gene fragments which encode leader signals that direct them to the periplasmic space of *E. coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty et al. (1990) Nature, 348: 552; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference). Similar techniques may be used for the generation of FASTbody libraries according to the present invention.

One particularly advantageous approach has been the use of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson et al. (1991) Nature, 352: 624; Marks et al. (1991) J. Mol. Biol., 222: 581; Chiswell et al. (1992) Trends Biotech., 10: 80; Marks et al. (1992) J. Biol. Chem., 267**).** Various embodiments of scFv libraries displayed on bacteriophage coat proteins have been described. Refinements of phage display approaches are also known, for example as described in WO96/06213 and WO92/01047 (Medical Research Council *et al*.) and WO97/08320 (Morphosys), which are incorporated herein by reference.

Other systems for generating libraries of polypeptides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/05258 and WO2/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO2/02536. Alternative display systems which are not phage-based, such as those disclosed in WO95/22625 and WO95/11922 (Affymax) use the polysomes to display polypeptides for selection.

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

### Library Construction.

Libraries intended for use in selection may be constructed using techniques known in the art, for example as set forth above, or may be purchased from commercial sources. Once a vector system is chosen and one or more nucleic acid sequences encoding polypeptides of interest are cloned into the library vector, one may generate diversity within the cloned molecules by undertaking mutagenesis prior to expression; alternatively, the encoded proteins may be expressed and selected, as described above, before mutagenesis and additional rounds of selection are performed. Mutagenesis of nucleic acid sequences encoding structurally optimised polypeptides is carried out by standard molecular methods. Of particular use is the polymerase chain reaction, or PCR, (Mullis and Faloona (1987) Methods Enzymol., 155: 335, herein incorporated by reference). PCR, which uses multiple cycles of DNA replication catalysed by a thermostable, DNA-dependent DNA polymerase to amplify the target sequence of interest, is well known in the art. The construction of various antibody libraries has been discussed in Winter et al. (1994) Ann. Rev. Immunology 12, 433-55, and references cited therein.

PCR is performed using template DNA (at least 1fg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. Mineral oil is overlaid and the PCR is performed using a programmable thermal cycler. The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenized, mismatch is required, at least in the first round of synthesis. The ability to optimise the stringency of primer annealing conditions is well within the knowledge of one of moderate skill in the art. An annealing temperature of between 30 °C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### FASTbody LIGANDS.

Fastbodies according to the present invention having a defined ligand binding specificity may be constructed and/or selected from libraries as described herein. Such FASTbodies may be generated using the methods described herein.

FASTbody ligands may be naturally occurring or synthetic. Naturally occurring ligands include antibodies, peptides, other proteins including for example hormones and signalling molecules and fatty acids.

As used herein the term 'fatty acid' includes within its scope fatty acid derivatives, homologues, analogues and/or fragments thereof so long as such derivatives, homologues, analogues and/or fragments thereof possess the requisite activity of FASTbody binding and the consequent modulation of specific ligand binding to a FASTbody as herein described.

In a preferred embodiment of the above aspect of the invention, the FASTbody according to the present invention is capable of specifically binding to RAS.

### Antibody preparation.

Either recombinant proteins or those derived from natural sources can be used to generate antibodies using standard techniques, well known to those in the field. For example, the protein (or "immunogen") is administered to challenge a mammal such as a monkey, goat, rabbit or mouse. The resulting antibodies can be collected as polyclonal sera, or antibody-producing cells from the challenged animal can be immortalized (e.g. by fusion with an immortalizing fusion partner to produce a hybridoma), which cells then produce monoclonal antibodies.

### a. Polyclonal antibodies

The antigen protein is either used alone or conjugated to a conventional carrier in order to increases its immunogenicity, and an antiserum to the peptide-carrier conjugate is raised in an animal, as described above. Coupling of a peptide to a carrier protein and immunizations may be performed as described (Dymecki et al. (1992) J. Biol. Chem., 267: 4815). The serum is titered against protein antigen by ELISA or alternatively by dot or spot blotting (Boersma and Van Leeuwen (1994) J. Neurosci. Methods, 51: 317). The serum is shown to react strongly with the appropriate peptides by ELISA, for example, following the procedures of Green et al. (1982) Cell, 28: 477.

### b. Monoclonal antibodies

Techniques for preparing monoclonal antibodies are well known, and monoclonal antibodies may be prepared using any candidate antigen, preferably bound to a carrier, as described by Arnheiter et al. (1981) Nature, 294, 278. Monoclonal antibodies are typically obtained from hybridoma tissue cultures or from ascites fluid obtained from animals into which the hybridoma tissue was introduced. Nevertheless, monoclonal antibodies may be described as being "raised against" or "induced by" a protein.

After being raised, monoclonal antibodies are tested for function and specificity by any of a number of means. Similar procedures can also be used to test recombinant antibodies produced by phage display or other *in vitro* selection technologies. Monoclonal antibody-producing hybridomas (or polyclonal sera) can be screened for antibody binding to the immunogen, as well. Particularly preferred immunological tests include enzyme-linked immunoassays (ELISA), immunoblotting and immunoprecipitation (see Voller, (1978) Diagnostic Horizons, 2: 1, Microbiological Associates Quarterly Publication, Walkersville, MD; Voller et al. (1978) J. Clin. Pathol., 31: 507; U.S. Reissue Pat. No. 31,006; UK Patent 2,019,408; Butler (1981) Methods Enzymol., 73: 482; Maggio, E. (ed.), (1980) Enzyme Immunoassay, CRC Press, Boca Raton, FL) or radioimmunoassays (RIA) (Weintraub, B., Principles of radioimmunoassays, Seventh Training Course on Radioligand Assay Techniques, The Endocrine Society, March 1986, pp. 1-5, 46-49 and 68-78), all to detect binding of the antibody to the immunogen against which it was raised. It will be apparent to one skilled in the art that either the antibody molecule or the immunogen must be labeled to facilitate such detection. Techniques for labeling antibody molecules are well known to those skilled in the art (see Harlour and Lane (1989) Antibodies, Cold Spring Harbor Laboratory, pp. 1-726).

Alternatively, other techniques can be used to detect binding to the immunogen, thereby confirming the integrity of the antibody which is to serve either as a generic antigen or a target antigen according to the invention. These include chromatographic methods such as SDS PAGE, isoelectric focusing, Western blotting, HPLC and capillary electrophoresis.

"Antibodies" are defined herein as constructions using the binding (variable) region of such antibodies, and other antibody modifications. Thus, an antibody useful in the invention may comprise whole antibodies, antibody fragments, polyfunctional antibody aggregates, or in general any substance comprising one or more specific binding sites from an antibody. The antibody fragments may be fragments such as Fv, Fab and F(ab')₂ fragments or any derivatives thereof, such as a single chain Fv fragments. The antibodies or antibody fragments may be non-recombinant, recombinant or humanized. The antibody may be of any immunoglobulin isotype, e.g., IgG, IgM, and so forth. In addition, aggregates, polymers, derivatives and conjugates of immunoglobulins or their fragments can be used where appropriate.

### REGULATION OF LIGAND BINDING TO A FASTbody ACCORDING TO THE INVENTION.

During experiments described above, the inventors found that the presence of one or more fatty acids bound to the FASTbody of the present invention regulates the specific binding of individual scaffolds to their ligand. Specifically they have found that the presence of one or more fatty acids selected from the group consisting of those fatty acids shown below in Table 2, in the fatty acid binding pocket of a FASTbody according to the invention regulates the specific binding of ligand to the scaffold.

As used herein the term 'fatty acid' includes within its scope fatty acid derivatives, homologues, analogues and/or fragments thereof so long as such derivatives, homologues, analogues and/or fragments thereof possess the requisite activity of FASTbody binding and the consequent modulation of specific ligand binding to a FASTbody as herein described.

**Table 2: Common Fatty Acids**

| Chemical Names and Descriptions of some Common Fatty Acids | | | | |
|---|---|---|---|---|
| **Common Atoms** | **Carbon Name** | **Double Bonds** | **Scientific Name** | **Sources** |
| Butyric acid 4 | 4 | 0 | butanoic acid | butterfat |
| Caproic Acid | 6 | 0 | hexanoic | acid butterfat |
| Caprylic Acid | 8 | 0 | octanoic acid | coconut oil |
| Capric Acid | 10 | 0 | decanoic acid | coconut oil |
| Lauric Acid | 12 | 0 | dodecanoic acid | coconut oil |
| Myristic Acid | 14 | 0 | tetradecanoic acid | palm kernel oil |
| Palmitic Acid | 16 | 0 | hexadecanoic | acid palm oil |
| Palmitoleic Acid | 16 | 1 | 9-hexadecenoic acid | animal fats |
| Stearic Acid | 18 | 0 | octadecanoic acid | animal fats |
| Oleic Acid | 18 | 1 | 9-octadecenoic acid | olive oil |
| Linoleic Acid | 18 | 2 | 9,12-octadecadienoic acid | corn oil |
| Alpha-Linolenic Acid | 18 | 3 | 9,12,15-octadecatrienoic acid | flaxseed (linseed) oil |
| Gamma-Linolenic Acid (GLA) | 18 | 3 | 6,9,12-octadecatrienoic acid | borage oil |
| Arachidic Acid | 20 | 0 | Eicosanoic acid | peanut oil, fish oil |
| Gadoleic Acid | 20 | 1 | 9-eicosenoic acid | fish oil |
| Arachidonic Acid (AA) | 20 | 4 | 5,8,11,14-eicosatetraenoic acid | liver fats |
| EPA | 20 | 5 | 5,8,11,14,17-eicosapentaenoic acid | fish oil |
| Behenic acid | 22 | 0 | docosanoic acid | rapeseed oil |
| Erucic acid | 22 | 1 | 13-docosenoic acid | rapeseed oil |
| DHA | 22 | 6 | 4,7,10,13,16,19-docosahexaenoic acid | fish oil |
| Lignoceric acid | 24 | 0 | tetracosanoic acid | small amounts in most fats |

Advantageously, the ligand is RAS. More advantageously, the ligand is RAS and the fatty acid is one or more of those listed above. More advantageously, the ligand is RAS and the fatty acid is Oleic Acid.

Thus according to the present invention, FASTbodies are contemplated wherein the affinity of ligand binding may be regulated by binding the FASTbody to the ligand in the presence of one or more fatty acids described herein. Further the measurement of ligand binding to the FASTbody can be performed by extrapolating the change in fatty acid dissociation to binding affinity of FASTbody to ligand.

### COMPARISION OF FASTbodies WITH ANTIBODIES

The above results indicate that the selected scaffold structure can be compared to antibodies, where the individual loop regions compare to the different CDR regions of the antibody, especially the 9 amino acid randomized peptide can be compared to the Heavy Chain CDR3 regions, since this position in the scaffold can accommodate significant variations in length and structure without disturbing the overall fold of the scaffold.

The novel feature of the present scaffold is the absence of disulphide bridge formation for stability, making it particularly well suited for intracellular targeting. The selected binder toward recombinant human RAS, was shown in competitive ELISA to compete the binding of RAF to RAS. As a first test of the in vivo intracellular effect, the binder was expressed intracellular in Mucor.

By growing Mucor expressing the binder under aerobic conditions phenotype of increased branching was observed. By Anaerobic growth of the same strains of mucor, inhibition of growth was observed. Both of these phenotypes previously has been observed in published experiments, in which the signal transduction pathway in which RAS takes part, is inhibited (Lübbehüsen T. et al. 2004). Taken together the novel features of the present inventions advantageously can be used to design validation systems for use in combination with other High Throughput screening systems to identify small chemical entities of therapeutic value. Details of such methods are described in the following patents and applications which are herein incorporated by reference: US6010861, US6617114, W09954728.

The invention will now be described in the following Examples which should not be considered limiting of the invention.

### Examples:

### Example 1

### Construction of phagemid vector encoding A-FABP.

The gene encoding the A-FABP (figure 4) was amplified using PCR. As template A-FABP in the eukaryotic expression vector pMT21 was used (Celis J.E. 1996).
The two primers FABPback and FABPfor were used in the amplification incooporating a Nco1 site at the N-Terminal part of FABP and an Not1 site at the C-terminal part. In addition the cystein present at the C-Terminal end of wt A-FABP was deleted..

The amplification of A-FABP were done using 25 pmol of each primer, 0,2 mM dNTP, 1,5 mM MgCl₂, 1xTaq polymerase buffer (20 mM Tris-HCl (pH 8.4), 50 mM KCl) and 2,5 units Taq DNA polymerase.

The amplification reaction were performed using the following temperature profile: 96°C 5 min; 20 x (96°C 30 s; 55°C 30 S; 72°C 1 min); 72°C 10 min.

The PCR product were purified using Qiagen PCR purification kit.

The vector pHEN2 and the PCR products were digested with the restrictions enzymes NcoI and NotI at 37°C overnight. Following gelpurification the A-FABP insert were ligated into to digested pHEN2 vector using T4 DNA ligase in a standard reaction (Figure 5).
The ligation were electroporated in TG-1 electrocompetent bacteria and plated on TYE/amp/glu plates.

Colonies were picked and sequenced using M13rev and M13Back.

Oligo:
FABPback: 
5′- ATGATGATGAGCGGCCGCTGCTCTCTCATAAACTCTCG – 3′
NotI FABP C-terminal
FABPfor

5’ – CAGCCGGCCATGGCCGATGCTTTTGTAGGTACCTGG – 3’
NcoI FABP N-Terminal

### Construction of phagemid vector encoding a helix-less A-FABP.

In the wild type sequence of the A-FABP a DNA recognition sequence for the restriction enzyme KpnI is situated 25 nucleotides before the beginning of the helix-loop-helix motif. In order to introduce unique restrictions sites at close distance at either site of the helix-loop-helix region and at the same time remove the helix-loop-helix motif a PCR reaction were performed essentially as described in Example 1 using the primer FABPback described previously and
Helix-minus-forward:

5′- ttgtaggtacctggaaacttgtctcgagtgaagctggcatgtccggacctaacatg - 3′

KpnI XhoI BstE1

The helix-minus-forward primer introduces unique XhoI and BstE1 sites.

The resulting PCR product were purified and cleaved using KpnI and NotI and ligated into pHEN2-aFABP vector (Figure 6). The ligation were electroporated in TG-1 and the sequence were verified by DNA sequencing using Primers M13 rev and M13 Back.

### Construction of a randomized 9 amino acid library in the helix-loop-helix region

To create a randomized 9 amino acid library, were the randomized region replaces the helix-loop-helix motif, the above described pHEN2 containing the helix-less A-FABP were digested with XhoI and BspEI.
The insert were constructed in a extension reaction where the synthetic oligo ran9helix were used as template in a single sited PCR. 
Ran9helix:5′-

TGGAAACTTGTCTCGAGTGAAAACNNKNNKNNKNNKNNKNNKNNKNNKNNKAGGTCCGGACCTAACATGAT

XhoI BspEI
Ampran9:
   5'- ATCATGTTAGGTCCGGACCT-3'

The amplification of randomized oligo were done using 25 pmol of each of the ran9helix oligo and ampran9, 0,2 mM dNTP, 1,5 mM MgCl₂, 1xTaq polymerase buffer (20 mM Tris-HCl (pH 8.4), 50 mM KCl) and 2,5 units Taq DNA polymerase. The amplification reaction were performed using the following temperature profile: 96°C 5 min; 10 x (96°C 30 s; 55°C 30 S; 72°C 1 min); 72°C 10 min
The PCR product were purified and digested using an excess of XhoI and BspEI in an overnight reaction after which the insert purified on a Microcon 100 to remove the ends of the Insert.

Ligation were performed using an insert to vector ratio of 10:1.
The ligation were phenol extracted and precipitated a standard sodium acetate/ethanol precipitation and dissolved in H₂O.

The purified ligation mixture were electroporated in TG-1 in 20 independent electroporations, which were mixed and plated on TYE/AMP/glu plates.

A 10 fold dilution series were made in order to determine the number or independent clones obtained.

The randomized 9 amino acid library (figure 7) contained 5x10⁶ to 1x10⁷ independent clones of which 20 were chosen at random for sequence verification.

The bacteria from the large TYE/AMP/glu plates were scarped by adding 3 ml 2xTY/AMP to each plate, thereby obtaining a suspension. The content from all the plates were pooled and glycerol to a final concentration of 15 % were added before storage in aliquots at -80°C.

### Rescue of the randomized 9 amino acid FASTbody library.

In order to present the FASTbody structures on the surface of filamentous bacteriophage the phagemid were rescued using a helper phage according to standard procedures.
1. 500 µl of the 9 amino acid FASTbody library stock were added to 200 ml 2xTY containing 100 µg/ml ampicillin and 1 % glucose.
2. Grow shaking at 37°C until the OD 600 is 0.5.
3. Add 1x10¹² KM13 helper phage (Kristensen P. 1998)
4. Incubate without shaking at 37°C for 45 min and with shaking at 37°C for 45 min.
5. Spin at 3,000 g for 10 min. Resuspend in 500 ml of 2xTY containing 100 □g/ml ampicillin, 50 µg/ml kanamycin.
6. Incubate shaking at 30°C overnight.
7. Spin the overnight culture at 3,300 g for 30 min.
8. Add 125 ml PEG/NaCl (20 % Polyethylene glycol 6000, 2.5 M NaCl) to 500 ml supernatant. Mix well and leave for 1 hr on ice.
9. Spin 3,300 g for 30 min. Pour away PEG/NaCl. Respin briefly and aspirate any remaining dregs of PEG/NaCl.
10. Resuspend the pellet in 40 ml PBS and spin at 11,600 g for 10 min in a micro centrifuge to remove any remaining bacterial debris.
11. Add 8 ml PEG/NaCl, mix and leave on ice for 1 hour)
12. Spin 3,300 g for 30 min. Pour away PEG/NaCl. Respin briefly and aspirate any remaining dregs of PEG/NaCl.
13. Resuspend the pellet in 20 ml PBS and spin at 11,600 g for 10 min in a micro centrifuge to remove any remaining bacterial debris.
14. Add glycerol to a final concentration of 15 % and store at - 80oC in small aliquots until use.
15. To titre the phage stock dilute 1µl phage in 100µl PBS, 1µl of this in 100µl PBS and so on until there are 6 dilutions in total. Add 900µl of TG1 at an OD 600 of 0.5to each tube and incubate at 37°C in a waterbath for 30 mins. Plate each dilution on a TYE plate containing 100 µg/ml ampicillin and 1 % glucose and grow overnight at 37°C. Phage stock should be 10¹²-10¹³/ml.

### Example 2

### Selection of the 9 amino acid FASTbody library on models proteins.

To examine the potential of generate binders from the 9 amino acid randomized FASTbody library, test selections were performed on the recombinant human RAS commercially available from sigma (R9894). The selection essentially followed standard procedures as described below:
1) Coat immunotube overnight with 4 ml 50 mM HCO₃ pH 9.6 containing 50 µg RAS.
2) Next day wash tube 3 times with PBS
3) Fill tube to brim with 2 % MPBS (2 % Skimmed milk powder in PBS). Incubate at rt. standing on the bench for 2 hr to block.
4) Wash tube 3 times with PBS.
5) Add 10¹² to 10¹³ phage from the phage displaying FASTbody library in 4 ml of 2 % MPBS. Incubate for 60 min at rt. rotating using an under-and-over turntable and then stand for a further 60 min at rt. Throw away supernatant.
6) Wash tubes 10 times with PBS containing 0.1 % Tween 20 and 10 times with PBS.
7) Shake out the excess PBS and elute phage by adding 500 µl of trypsin-PBS (50 µl of 10mg/ml trypsin stock solution added to 450 µl PBS) and rotating for 10 min at rt using an under-and-over turntable.
8) Take 10 ml of TG1 at an OD 600 of 0.5 and add the eluted phage. Incubate for 30 min at 37°C without shaking.
9) Make 10 fold dilutions and plate these of TYE plates containing 100 µg/ml ampicillin and 1 % glucose. The remaining bacteria is collected by centrifugation at 3,000 g for 10 min, the pellet is dissolved in 200-500 µl media and plated on TYE plates containing 100 µg/ml ampicillin and 1 % glucose.
10) Grow plates at 37°C overnight.

### Monoclonal phage ELISA for binding to RAS:

In order to identify individual colonies producing phage particles expressing the FASTbody structure which recognize RAS ELISA is performed:

### Rescue

1. Inoculate individual colonies from the plates from the first round of selection into 100 µl 2xTY containing 100 µg/ml ampicillin and 1 % glucose in 96 cell-well plates. Grow shaking (250 rpm) overnight at 37°C.
2. Use a 96 well transfer device to transfer a small inoculum from this plate to a second 96 cell-well plate containing 200 µl of 2xTY with 100 µg/ml ampicillin and 1 % glucose per well. Grow shaking (250 rpm) at 37°C for 2 hr. (Make glycerol stocks of the original 96-well plate, by adding glycerol to a final concentration of 15 %, and then storing the plates at -80°C).
3. After 2 hr incubation add 25 µl 2xTY containing 100 µg/ml ampicillin, 1 % glucose and 10⁹ KM13 helper phage (Kristensen P. 1998).
4. Shake (250 rpm) at 37°C for 1 hr. Spin 1,800 g for 10 min, then shake out the supernatant.
5. Resuspend pellet in 200 µl 2xTY containing 100 µg/ml ampicillin and 50 µg/ml kanamycin. Grow shaking (250 rpm) overnight at 30°C.
6. Spin at 1,800 g for 10 min and use 50 µl of the supernatant in phage ELISA.

### ELISA

7. A 96 well NUNC maxisorp plate is coated overnight with 100 µl 50 mM HCO₃ pH 9.6 containing 50 ng RAS per well. In addition two plates are coated with non-relevant antigens (ubiquitin and BSA) for checking cross reactivity toward other antigen. Also a plate is left empty controlling for phage carrying a scaffold binding to the blocking agent. All plates are treated similarly as indicated below
8. Wash wells 3 times with PBS.
9. Add 300 µl per well of 2 % MPBS (2 % Skimmed milk powder in PBS) to block and incubate for 2 hr at room temperature.
10. Wash wells 3 times with PBS. Add 50 µl phage supernatant from above and 50 µl 4 % MPBS.
11. Incubate for 2 hr at room temperature shaking. Discard phage solution and wash 3 times with PBS-0.1 % Tween 20 and 3 times with PBS.
12. Add 1 in 5000 dilution of HRP-anti-M1311 in 2 % MPBS 100 µl per well Incubate for 1 hr at room temperature.
13. Wash 3 times with PBS-0.1 % Tween 20 and 3 times with PBS
14. Add 100 µl of substrate solution (4 OPD tablets in 12 ml H2O and 50 ml 30 % H₂O₂).
15. Stop the reaction by adding 50 µl 1 M sulphuric acid.
16. Read the OD at 495 nm

### Results.

As described above 96 colonies were tested for binding to RAS, Ubiqutin, BSA and skimmed milk powder in PBS. A number of positive clones were isolated, below the binding of 4 clones to the 4 antigens is represented.
The three clones 2-4-F; 3-12-F; 3-11-E showed specific binding to RAS (Figure 8).

As the production of phage particles took place by growth in a yeast extract it was considered a possibility that the FASTbodies potentially could be binding a Fatty Acid at the same time as binding to the target RAS.

Since BSA naturally binds fatty acids, an ELISA experiment was performed in which the Skimmed milk powder used in the blocking and incubation steps described in the procedure above, was replaced with 2% BSA. If the FASTbody binds fatty acids and this binding is necessary for binding to the target RAS, a decreased binding to the target RAS would be expected in the presence of BSA. Figure 9 show the result of the ELISA, especially for the clones 2-4-F and 3-11-E a decreased specific binding to RAS is observed, thus indicating that the binding to RAS is influenced by the presence of fatty acids in the FASTbodies.

### Example 3

Based on the one of the clones isolated for binding to RAS (3-11-E from Example 2, see figure 10 for sequence information) a new library were constructed by randomizing to loop connecting β-strand E and F.

In order to allow easy manipulation of the loop between β-strand E and F a unique restriction site was introduced following the loop sequence. Together with a unique PstI restriction site presence in front of the loop sequence, this allows for easy manipulation of the loop region.

The two primers β5β6SerS and FABP545AS were used in the amplification The primer β5β6SerS incooporates a XbaI site following the loop sequence and at the same time carries the PstI restriction site allowing insertion in the backbone of FABP. The primer FABP545AS is located 142 nucleotides downstream of the NotI site. 
β5β6 SerS: 5'-
GTCACTGCAGATGACTCTAGAAAAGTCAAGAGCACCATAACC- 3'
FABP 545 AS : 5'-TTGTCGTCTTTCCAGACGTTAG-3'

The amplification were done using 25 pmol of each primer, 0,2 mM dNTP, 1xPfu polymerase buffer (20 mM Tris-HCl (pH 8.4), 50 mM KCl, 1,5 mM MgCl₂) and 2,5 units Pfu DNA polymerase. As template phagemid DNA from the clone 3-11-E was used. The amplification reaction were performed using the following temperature profile: 96°C 5 min; 20 x (96°C 30 s; 55°C 30 S; 72°C 1 min); 72°C 10 min. The PCR product of 329 base pair was purified and digested with the restriction enzymes PstI and NotI using standard conditions and ligated into the phagemid encoding the binder 3-11-E.

Two different libraries were generated by inserting 5 and 7 randomised amino acid in the loop connecting β-E and β-F.

Two PCR reactions were performed using standard conditions as outlined above Figure 11.
The first primary PCR used the primers FABP 61S and β5β6 AS, amplifying a fragment starting around the HindIII site in the vector sequence and finishing in front of the loop between β-strand E and F.
The second primary PCR used the primers FABP545AS and either b5b6S R5 or b5b6S R7 (introducing 5 and 7 randomised residues respectively), amplifying a fragment covering the loop to be randomized and finishing in the vector sequence.

Primers used:
β5β6 AS: AGT GAC TTC GTC AAA TTC C 
β5β6 S 7R: GGA ATT TGA CGA AGT CAC TNN KNN KNN KNN KNN KNN
KNN KAG GAA AGT CAA GAG CAC C
 
β5β6 S 5R: GGA ATT TGA CGA AGT CAC TNN KNN KNN KNN KNN KAG
GAA AGT CAA GAG CAC C
FABP 545 AS : 5'-TTGTCGTCTTTCCAGACGTTAG-3'
FABP 61 S : AAT GAA ATA CCT ATT GCC TAC GG

The PCR products were purified using Qiagen PCR prep kit according to instructions. The PCR fragments were assembled in a secondary PCR reaction using the same outside primers as above (FABP 61S and FABP 545AS) in standard PCR conditions. The PCR product were gel-purified and digested with the restriction enzymes Pst1 and Not1 (using standard procedures as outlined above). Followed by ligation into the vector encoding the 3-11-E FASTbody previously digested with the same enzymes and purified.

### Example 4

The second generation library based on the binder 3-11-E to RAS, where the loop region connecting β-E and β-F had been replaced with either 5 or 7 randomised amino acids was selected for binding to RAS as described in example 2.
Following selection 96 colonies were tested for binding to RAS using a similar procedure as described above in example 2.

For 5 of the FASTbodies giving a stronger signal compared to the parent FASTbody 3-11-E, the influence of adding fatty acids during the binding and washing steps were further examined.
1. A 96 well NUNC maxisorp plate is coated overnight by adding 100 µl 2 % skimmed milk powder in PBS in the first row. In the second row 100 µl 50 mM HCO₃ pH 9.6 containing 50 ng RAS per well. In the third row 100 µl 2 % skimmed milk powder and 100 µM Oleate. In the fourth row 100 µl 50 mM HCO₃ pH 9.6 containing 50 ng RAS per well and 100 µM Oleate. The fifth row 100 µl 2% skimmed milk powder and 100 mM Oleate. And finally in the sixth row 100 µl 50 mM HCO₃ pH 9.6 containing 50 ng RAS per well and 100 µM Oleate.
2. Wash wells 3 times with PBS. The PBS used to wash row 5 and 6 in addition contained 100 µM Oleate.
3. Add 300 µl per well of 2 % MPBS (2 % Skimmed milk powder in PBS) to block and incubate for 2 hr at room temperature. The blocking solution used for row 5 and 6 contained 100 µM Oleate.
4. Wash wells 3 times with PBS again washing row 5 and 6 with PBS containing 100 µM Oleate Add 50 µl phage supernatant from above and 50 µl 4 % MPBS, adding Oleate to 100 µM in row 5 and 6.
5. Incubate for 2 hr at room temperature shaking. Discard phage solution and wash 3 times with PBS-0.1 % Tween 20 and 3 times with PBS. Again the buffers used for row 5 and 6 contained 100 µM Oleate
6. Add 1 in 5000 dilution of HRP-anti-M1311 in 2 % MPBS 100 µl per well Incubate for 1 hr at room temperature, incubating row 5 and 6 in the presence of 100 µM Oleate
7. Wash 3 times with PBS-0.1 % Tween 20 and 3 times with PBS with 100 µM Oleate added to the washing solutions for row 5 and 6
8. Add 100 µl of substrate solution (4 OPD tablets in 12 ml H2O and 50 ml 30 % H₂O₂).
9. Stop the reaction by adding 50 µl 1 M sulphuric acid.
10. Read the OD at 495 nm

The results of the ELISA is shown in figure 12 and indicate clearly the randomization of the loop connecting b-strand E and F results in FASTbodies of with increased binding ability to RAS used in the selection. For some of the binders there is a clear influence on the addition of Oleate in the ELISA experiment, thus establishing that the binding of FASTbodies to the ligand is modulated by the presence of free fatty acids in the experiment.

All of the clones were further analysed by DNA sequencing using standard procedure. The loop sequences of the isolated FASTbodies is shown in Figure 13. All of the sequences are different and binders can be obtained both from the 5 amino acids randomized library and the 7 amino acid randomized library.

### Example 5

To allow testing of the binding properties of the isolated binders, the binders was cloned into the expression vector pET-11d.
The expression vector pET-11d previously had been modified to contain a NotI side followed by a myc-tag and a his-tag, thus allowing the binder to be cloned as a NcoI/NotI fragment (figure 13)
The FASTbodies can be subcloned into the modified pBT-11d vector by digesting the pHEN2 vector carrying the FASTbody with the restriction enzymes NcoI and NotI. The DNA fragment is the gel-purified and ligated into the modified pET-11d vector previously digested with the same enzymes. The ligation follows standard protocols. The Ligation is the transformed into E.Coli stains with a T7 DNA polymerase under control of a lacZ promoter, such as ER2566 (novagen).

### Expression of FABP-binders:

Clones were picked from TYE-plates, grown overnight at 37°C in 2xTY supplemented with 100µg/mL ampicillin and 1 % glucose, before dilution 1:100 into 2xTY with 100µg/mL ampicillin and 0,1 % glucose and incubated for four hours at 37°C shaking (OD600 should be around 0.7-0.9). The cultures were induced by addition of 1mM IPTG and grown overnight at room temperature. Cells were pelleted at 6000xg and resuspended in 50mM NaₓH_{y}PO₄ pH 8,0 before lysis in French Press (American Instruments Co., inc. Silver Spring, MD, USA) . The suspension was subsequently cleared by centrifugation (26000xg) and the supernatant supplemented with 30mM Imidazole and 300mM NaCl before being subjected to immobilised metal affinity chromatography (IMAC). Ni-NTA was incubated with the supernatant for 2 hours at 4°C, and was subsequently washed with a minimum of 100 mL wash buffer (50 mM NaₓH_{y}PO₄ pH 8,0, 300 mM NaCl, 30 mM Imidazole) followed by 50 mL of high saline wash buffer (50 mM NaₓH_{y}PO₄ pH 8,0, 750 mM NaCl, 30 mM Imidazole). Protein was eluted with wash buffer supplemented with Imidazole to 300 mM. Protein concentration was determined according to Bradford and the purity analysed by sodium dodecyl sulphate polyacryl amide gel electrophoresis (SDS-PAGE).

### Example 6

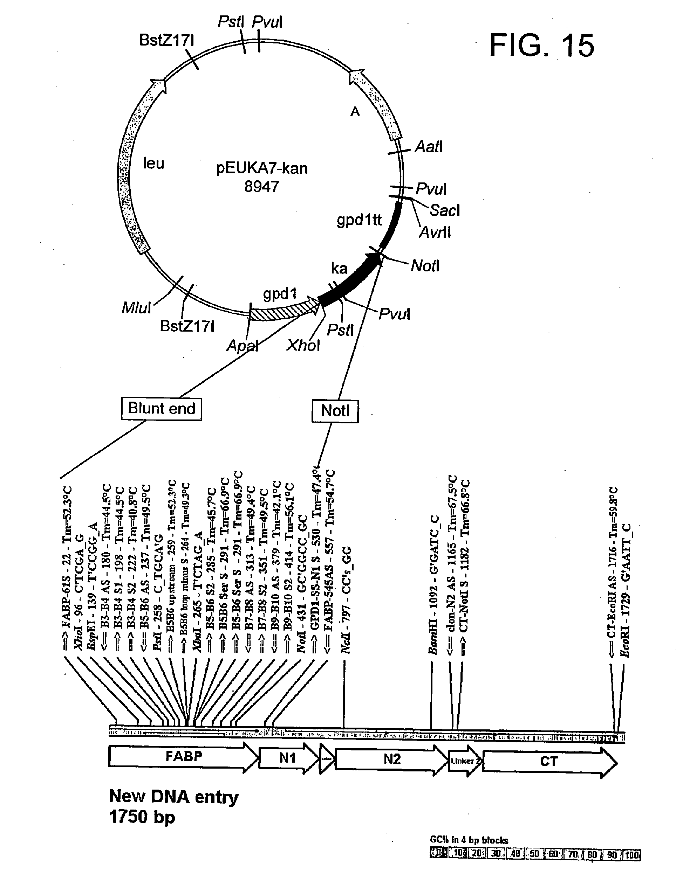

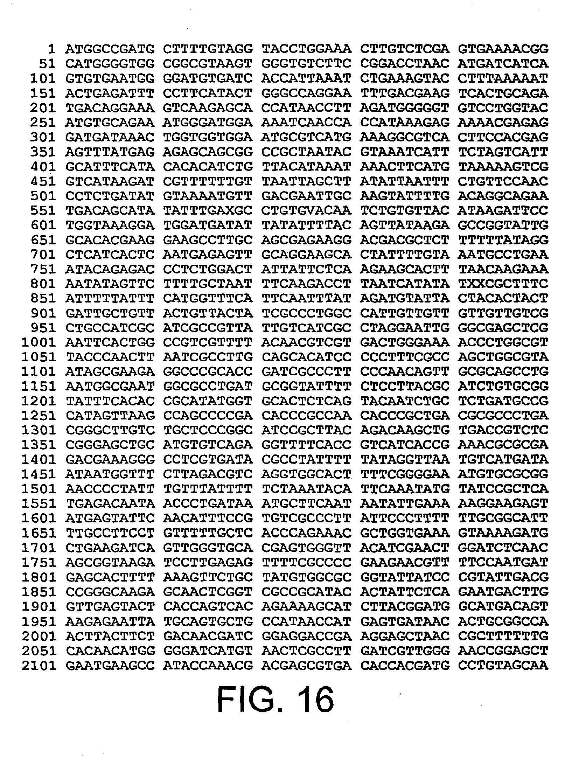

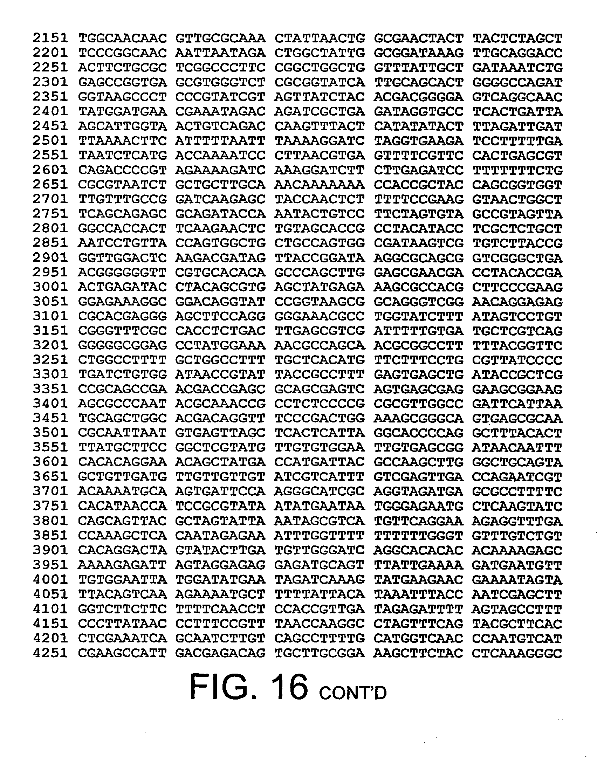

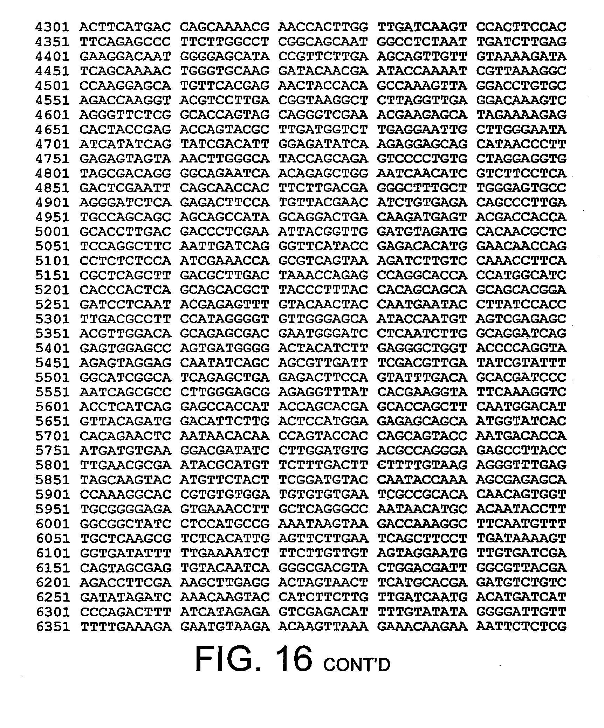

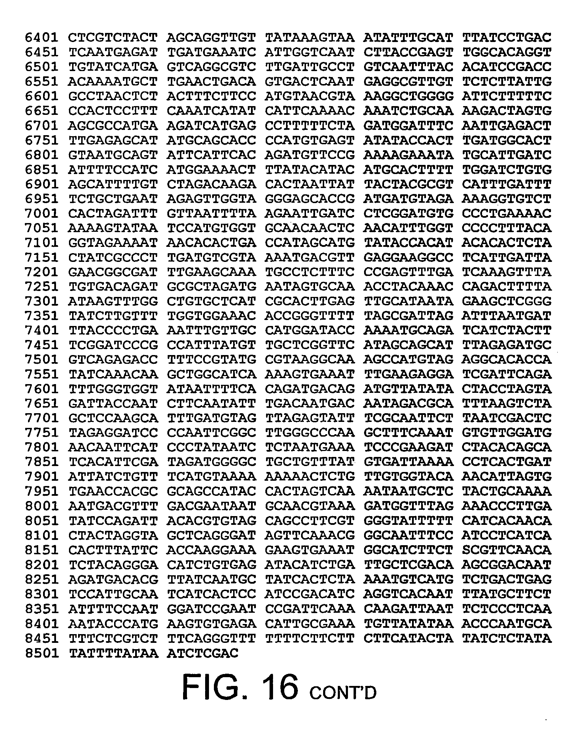

To establish the biological activity of expressing a FASTbody binding to RAS intracellular the RAS binding (Appel K.F. et al 2004 and figure 15 and figure 16) by digesting the pHEN2 vector carrying the FASTbody with the restriction enzyme NcoI according to 3-11-E 89-8-E were introduced into the vector pEUKA7-kan standard procedures as described above. The linearised plasmid were treated with T4 DNA polymerase to create a blunt end according to manufactures instruction. Following this the DNA fragment encoding the FASTbody was generated by cleaving with the restriction enzyme NotI in a standard reaction and the DNA fragment was gel purified before ligation into the pEUKA7-Kan vector

### Transformation of M. circinelloides

Protoplasts formation and transformation were performed as previously described (Appel K.F.et al. 2004) with the following modifications. Protoplasts were prepared by enzymatic treatment of germlings with a mixture of 125 µg chitosanase-RD (US Biological, MA, USA) and 5 U chitinase (from Streptomyces griseus, Sigma) in a final volume of 2 ml. Cell wall digestion was carried out for 2-3 h at 28°C. Typically, 1-10 µg DNA was used per transformation. Transformants were selected on YNBmedium . Mucor transformant strains KFA143 in which the FASTbody is replaced by the kanamycin gene served as a control.

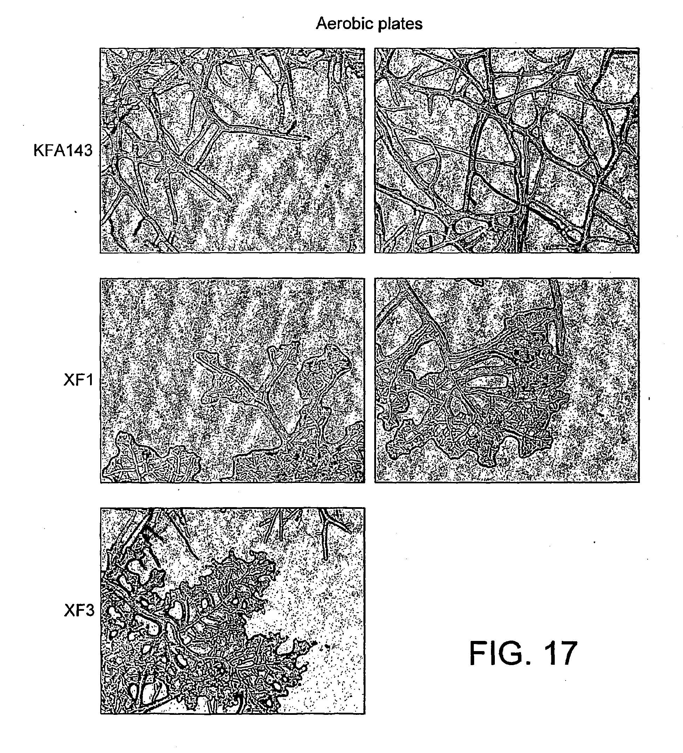

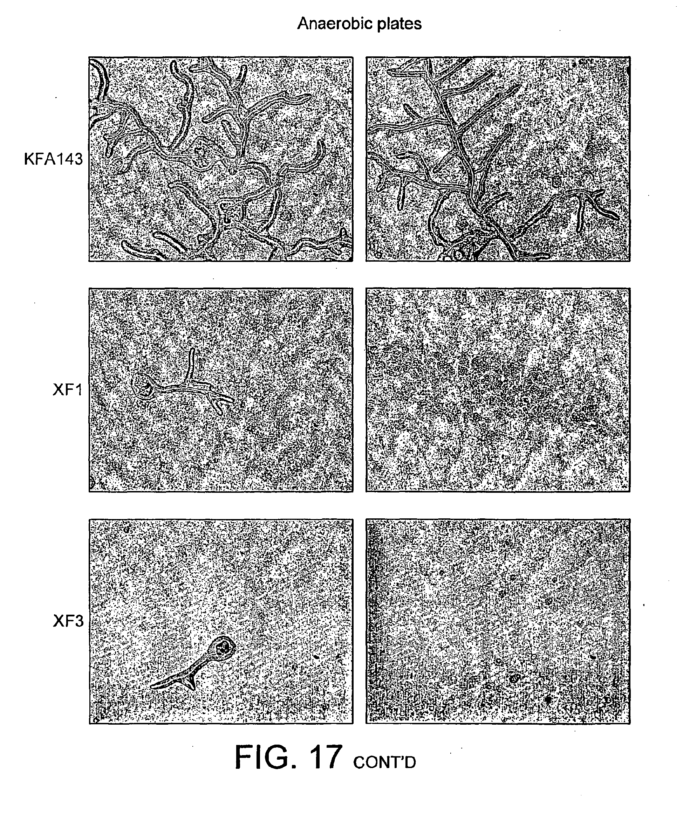

Transformed mucor strains were grown under aerobic or anaerobic conditions respectively (Figure 17). When mucor were grown under aerobic conditions markedly increased branching was observed, a phenotype resembling the phenotypes others have obtained when expressing mucor in which the RAS gene has been mutated. When grown under anaerobic conditions little or no growth was observed, also in line with previous studies from others. In conclusion there is a marked effect on phenotype when expressing FASTbodies binding to the RAS proteins intracellulary in mucor.

### Example 7

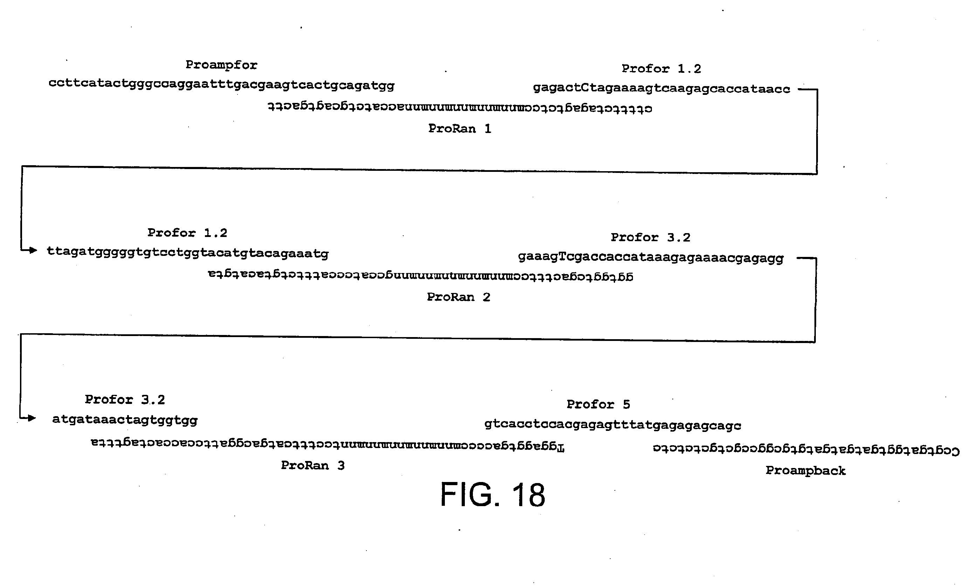

A library of synthetic gene fragments encoding the regions from the PstI site and to the NotI side of the FASTbody were created by oligo assembly as described below. The library of synthetic gene fragments result in randomization of the loop regions connecting β-strand E-F, β-stand G-H, β-strand I-J (figure 18). The library of synthetic gene fragments were then combined with the library described in Example 1, together creating a fully randomized FASTbody library.
The 8 synthetic oligo's Proampfor; ProRan1; Profor 1.2; ProRan 2; Profor 3.2; ProRan3; Profor 5 and Proampback were assembled by performing 10 assembly reactions each containing 10 pmol of each oligo and 0,2 mM dNTP, 1,5 mM MgCl₂, 1xTaq polymerase buffer (20 mM Tris-HCl (pH 8.4), 50 mM KCl) and 2,5 units Taq DNA polymerase. The assembly reactions were incubated with the following temperature profile for 5 cycles (96°C 30 s; 50°C 30 S; 72°C 1 min) followed by incubation at 72°C for 10 min. In order to amplify the assembled gene fragments 25 pmol of each of the primers Proampfor and Proampback were added to each reaction and amplification were performed in 10 cycles with the following profile (96°C 30 s; 55°C 30 S; 72°C 1 min) and finally extended at 72°C for 10 min.
The resulting genefragments were gel-purified using the Qiagen gel-purification kit and digested with the restriction enzymes PstI and NotI using standard conditions.
Plasmid from the FASTbody library prepared as described in example 1 were purified from 200 ml TG-1 culture using the Qiagen Midi-plasmid prep kit according to the manufactures instructions and the plasmid were digested using PstI and NotI using standard procedures. The pHEN2 vector containing part of the FASTbody gene carrying the randomized 9 amino acid sequence were gelepurified and optimatisation of ligation reactions were carried out using T4 DNA ligase under standard condition. The resulting ligated total randomized FASTbody library were electroporated in TG-1 and the library were rescued essentially as described in example 1 for the 9 amino acid library, resulting in a FASTbody library containing 2x10⁷ different sequences as estimated from the number of clones obtained from the ligation reaction.

Proampfor
ccttcatactgggccaggaatttgacgaagtcactgcagatgg

ProRan 1
cttttctagagtctccmnnmnnmnnmnnmnnaccatctgcagtgactt

Profor 1.2
gagactCtagaaaagtcaagagcaccataaccttagatgggggtgtcctggtacatgtacagaaatg

ProRan 2
Ggtggtcgactttccmnnmnnmnnmnnmnngccatcccatttctgtacatgta

Profor 3.2
gaaagTcgaccaccataaagagaaaacgagaggatgataaactagtggtgc

ProRan 3
Tggaggtgaccccmnnmnnmnnmnnmnntcctttcatgacggattccaccactagttta

profor 5
Gtcacctccacgagagtttatgagagagcagc

Proampback
Ccgtgatggtgatgatgatgtgcggccgctgctctctc

### Example 8

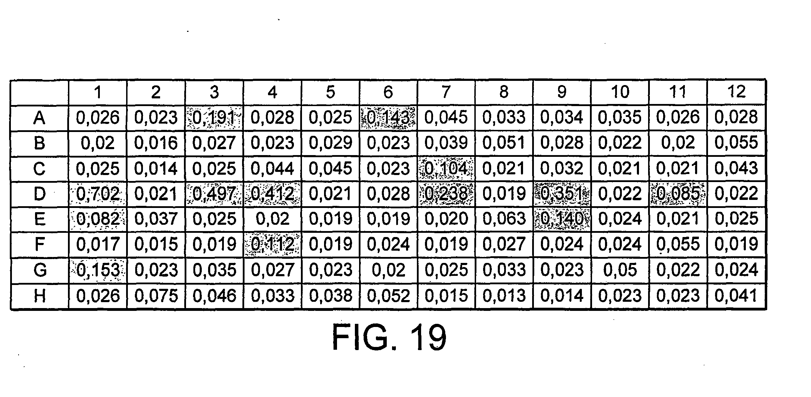

The total randomised FASTbody library described in example 7 were used to select binders against RAS as described in example 2 with the only modification being that 0,1 mg/ml Oleate were added to all buffers.
2x10⁵ colonies were obtained after the selection of which 96 were picked and a monoclonal rescue were performed in order to perform ELISA as described in Example 2 with the only modification the 0,1 mg/ml Oleate were added to all buffers. As shown in figure 19 there were at least 13 positive clones when a score above 0,08 as measure by absorbance at 490 mn were taken as positive. been described in connection with specific preferred embodiments, it should be understood that the invention as claimed should not be unduly limited to such specific embodiments.

## Claims

1. A fatty acid binding protein scaffold, designated a FASTbody, capable of specific binding to one or more ligands, which scaffold is derived from a eukaryotic intracellular fatty acid binding protein and which scaffold comprises a single-chain polypeptide with the following structural properties:
(a) The scaffold contains 10 β-strands, designated ABCDEFGHI and J, connected by loop regions which determine the specificity of ligand binding, wherein the β-strands together form a β-clam structure; and wherein
(b) The loop regions connecting β-A and β-B; β-C and β-D; β-E and β-F; β-G and β-H; β-I and β-J are located on the same site of the β-clam structure; wherein the fatty acid binding scaffold does not contain any disulphide bridge forming cysteines; wherein the scaffold does not comprise a helix-loop-helix motif.

2. A fatty acid binding scaffold according to claim 1 which consists of a single-chain polypeptide with the following structural properties:
(a) The scaffold contains 10 β-strands, designated ABDEFGHI and J, connected by loop regions which determine the specificity of ligand binding, wherein the β-strands together form a β-clam structure; and wherein
(b) The loop regions connecting β-A and β-B; β-C and β-D; β-E and β-F; β-G and β-H; β-I and β-J are located on the same site of the β-clam structure; wherein the fatty acid binding scaffold does not contain any disulphide bridge forming cysteines; wherein the scaffold does not comprise a helix-loop-helix motif.

3. A method for generating a fatty acid binding protein scaffold as defined in claim 1, designated a FASTbody, which is derived from a eukaryotic intracellular protein and which method comprises:
(a) Providing a single polypeptide chain fatty acid binding protein, or the nucleic acid encoding it; and
(b) Randomizing the helix-loop-helix motif, or the nucleic acid encoding it.

4. A method according to claim 3 which comprises a further step (c), wherein one or more loop regions connecting β-strands designated A, B, C, D, E, F, G, H, I and/or J, or the nucleic acid encoding them is randomized.

5. A method according to claim 4 wherein the loop region connecting β-strands B and E is randomized.

6. A method according to claim 4 wherein the loop regions connecting β-E and β-F, β-G and β-H and β-I and β-J are randomized.

7. A fatty acid binding protein scaffold, designated a FASTbody, which is derived from a eukaryotic intracellular protein obtainable according to the method of any of claims 4 to 6.

8. A nucleic acid construct encoding a fatty acid binding protein scaffold, designated a FASTbody, according to claim 1 or claim 2.

9. A vector comprising a nucleic acid construct according to claim 8.

10. A host cell comprising a vector according to claim 9.

11. A method for changing the ligand binding specificity of a fatty acid binding scaffold, designated a FASTbody, according to any of claims 1 to 7, which method comprises the steps of:
(a) Providing a fatty acid binding scaffold according to claim 1 or claim 2, and
(b) Randomizing one or more loops connecting the β-strands designated A, B, C, D, E, F, G, H, I and J.

12. A library of fatty acid binding protein scaffolds, designated FASTbodies, according to claim 1 or claim 2.

13. A method for selecting a fatty acid binding scaffold according to any of claims 1 to 7 which scaffold is capable of binding to a defined ligand which method comprises the steps of:
(a) Providing a fatty acid binding protein, designated a FASTbody, library according to claim 12.
(b) Testing the ability of the library according to step (a) to bind the defined ligand; and
(c) Selecting those fatty acid binding scaffolds according to step (b) which are capable of binding to the defined ligand.

14. The use of a fatty acid in modulating the binding of specific ligand to one or more FASTbodies according to any of claims 1 and 2.

15. The use of a fatty acid in the monitoring of the binding of specific ligands to one or more FASTbodies according to any of claims 1 and 2.

16. A method of identifying a potential drug candidate which is capable of displacing the binding of a FASTbody according to any of claims 1 to 7 to the target, which method comprises the steps of:
(a) Providing a library of FASTbodies according to claim 12,
(b) Providing a sample of one or more drug candidates; and
(c) Screening the library for the ability of the one or more drug candidates to displace the binding of one or more FASTbodies within the library according to step (a).

## Patentansprüche

1. Fettsäurebindungsproteingerüst, bezeichnet als FASTbody, welches zur spezifischen Bindung an einen oder mehrere Liganden in der Lage ist, wobei das Gerüst von einem eukaryotischen intrazellulären Fettsäurebindungsprotein abgeleitet ist und ein Einzelketten-Polypeptid mit den folgenden strukturellen Eigenschaften umfaßt:
(a) das Gerüst enthält 10 β-Stränge, bezeichnet mit ABCDEFGHI und J, die durch Schleifenregionen verbunden sind, welche die Spezifität der Ligandenbindung bestimmen, wobei die β-Stränge zusammen eine β-Muschelstruktur bilden, und wobei
(b) die Schleifenregionen, die β-A und β-B, β-C und β-D, β-E und β-F, β-G und β-H, β-I und β-J verbinden, an der gleichen Stelle der β-Muschelstruktur liegen, wobei das Fettsäurebindungsgerüst keine Disulfidbrücken bildenden Cysteine enthält, wobei das Gerüst kein Helix-Schleife-Helix-Motiv umfaßt.

2. Fettsäurebindungsgerüst nach Anspruch 1, welches aus einem Einzelketten-Polypeptid mit den folgenden strukturellen Eigenschaften besteht:
(a) das Gerüst enthält 10 β-Stränge, bezeichnet mit ABCDEFGHI und J, die durch Schleifenregionen verbunden sind, welche die Spezifität der Ligandenbindung bestimmen, wobei die β-Stränge zusammen eine β-Muschelstruktur bilden, und wobei
(b) die Schleifenregionen, die β-A und β-B, β-C und β-D, β-E und β-F, β-G und β-H, β-I und β-J verbinden, an der gleichen Stelle der β-Muschelstruktur liegen, wobei das Fettsäurebindungsgerüst keine Disulfidbrücken bildenden Cysteine enthält, wobei das Gerüst kein Helix-Schleife-Helix-Motiv umfaßt.

3. Verfahren zum Erzeugen eines Fettsäurebindungsproteingerüsts wie in Anspruch 1 definiert, bezeichnet als FASTbody, welches von einem eukaryotischen intrazellulären Protein abgeleitet ist und wobei das Verfahren folgendes umfaßt:
(a) Bereitstellen eines Fettsäurebindungsproteins mit einer einzelnen Polypeptidkette oder der dieses codierenden Nukleinsäure und
(b) Randomisieren des Helix-Schleife-Helix-Motivs oder der dieses codierenden Nukleinsäure.

4. Verfahren nach Anspruch 3, welches eine weitere Stufe (c) umfaßt, bei der eine oder mehrere Schleifenregionen, die mit A, B, C, D, E, F, G, H, i und/oder J bezeichnete β-Stränge verbinden, oder die diese codierende Nukleinsäure randomisiert wird bzw. werden.

5. Verfahren nach Anspruch 4, wobei die Schleifenregion, welche die β-Stränge B und E verbindet, randomisiert wird.

6. Verfahren nach Anspruch 4, wobei die Schleifenregionen, die β-E und β-F, β-G und β-H und β-I und β-J verbinden, randomisiert werden.

7. Fettsäurebindungsproteingerüst, bezeichnet als FASTbody, welches von einem eukaryotischen intrazellulären Protein abgeleitet ist und gemäß dem Verfahren nach einem der Ansprüche 4 bis 6 erhältlich ist.

8. Nukleinsäurekonstrukt, welches ein Fettsäurebindungsproteingerüst, bezeichnet als FASTbody, nach Anspruch 1 oder Anspruch 2 codiert.

9. Vektor, welcher ein Nukleinsäurekonstrukt nach Anspruch 8 umfaßt.

10. Wirtszelle, welche einen Vektor nach Anspruch 9 umfaßt.

11. Verfahren zum Verändern der Liganden-Bindungsspezifität eines Fettsäurebindungsgerüsts, bezeichnet als FASTbody, nach einem der Ansprüche 1 bis 7, wobei das Verfahren die folgenden Stufen umfaßt:
(a) Bereitstellen eines Fettsäurebindungsgerüsts nach Anspruch 1 oder Anspruch 2 und
(b) Randomisieren einer oder mehrerer Schleifen, die die mit A, B, C, D, E, F, G, H, I und J bezeichneten β-Stränge verbinden.

12. Bibliothek von Fettsäurebindungsproteingerüsten, bezeichnet als FASTbodies, nach Anspruch 1 oder Anspruch 2.

13. Verfahren zum Auswählen eines Fettsäurebindungsgerüsts nach einem der Ansprüche 1 bis 7, wobei das Gerüst an einen definierten Liganden binden kann und das Verfahren die folgenden Stufen umfaßt:
(a) Bereitstellen einer Bibliothek von Fettsäurebindungsprotein, bezeichnet als FASTbody, nach Anspruch 12,
(b) Testen der Fähigkeit der Bibliothek aus Stufe (a) zur Bindung des definierten Liganden und
(c) Auswählen derjenigen Fettsäurebindungsgerüste aus Stufe (b), die zur Bindung des definierten Liganden in der Lage sind.

14. Verwendung einer Fettsäure beim Modulieren der Bindung eines spezifischen Liganden an einen oder mehrere FASTbodies nach einem der Ansprüche 1 und 2.

15. Verwendung einer Fettsäure bei der Überwachung der Bindung spezifischer Liganden an einen oder mehrere FASTbodies nach einem der Ansprüche 1 und 2.

16. Verfahren zum Identifizieren eines potentiellen Arzneimittelkandidaten, welcher die Bindung eines FASTbody nach einem der Ansprüche 1 bis 7 an das Ziel verlagern kann, wobei das Verfahren die folgenden Stufen umfaßt:
(a) Bereitstellen einer Bibliothek von FASTbodies nach Anspruch 12,
(b) Bereitstellen einer Probe von einem oder mehreren Arzneimittelkandidaten und
(c) Screenen der Bibliothek hinsichtlich der Fähigkeit des einen oder der mehreren Arzneimittelkandidaten zur Verlagerung der Bindung eines oder mehrerer FASTbodies innerhalb der Bibliothek gemäß Stufe (a).

## Revendications

1. Molécule d'ossature de protéine de liaison aux acides gras, désignée sous le nom FASTbody, apte à la liaison spécifique à un ou plusieurs ligands, molécule d'ossature qui est dérivée d'une protéine intracellulaire eucaryotique de liaison aux acides gras et molécule d'ossature qui comprend un polypeptide monocaténaire ayant les propriétés structurales suivantes :
a) la molécule d'ossature contient 10 brins β, appelés ABCDEFGHI et J, connectés par des régions de boucle qui déterminent la spécificité de liaison de ligand, les brins β formant ensemble une structure de coquille β ; et où
(b) les régions de boucle connectant β-A et β-B, β-C et β-D, β-E et β-F, β-G et β-H, β-I et β-J sont situées sur le même site de la structure de coquille β ; la molécule d'ossature de liaison aux acides gras ne contenant aucun résidu cystéine formant un pont disulfure ; la molécule d'ossature ne comprenant pas de motif hélice-boucle-hélice.

2. Molécule d'ossature de liaison aux acides gras suivant la revendication 1, qui consiste en un polypeptide monocaténaire ayant les propriétés structurales suivantes :
(a) la molécule d'ossature contient 10 brins β, appelés ABDEFGHI et J, connectés par des régions de boucle qui déterminent la spécificité de liaison de ligand, les brins β formant ensemble une structure de coquille β ; et où
(b) les régions de boucle connectant β-A et β-B, β-C et β-D, β-E et β-F, β-G et β-H, β-I et β-J sont situées sur le même site de la structure de coquille β ; la molécule d'ossature de liaison aux acides gras ne contenant aucun résidu cystéine formant un pont disulfure ; la molécule d'ossature ne comprenant pas de motif hélice-boucle-hélice.

3. Procédé pour engendrer une molécule d'ossature de protéine de liaison aux acides gras telle que définie dans la revendication 1, désignée sous le nom de FASTbody, qui est dérivée d'une protéine intracellulaire eucaryotique, procédé qui comprend les étapes consistant :
(a) à fournir une protéine à chaîne polypeptidique unique de liaison aux acides gras, ou bien l'acide nucléique codant pour celle-ci ; et
(b) à randomiser le motif hélice-boucle-hélice, ou l'acide nucléique codant pour celui-ci.

4. Procédé suivant la revendication 3, qui comprend une étape supplémentaire (c), dans laquelle une ou plusieurs régions de boucle connectant les brins β désignés par A, B, C, D, E, F, G, H, I et/ou J, ou l'acide nucléique codant pour celles-ci sont randomisés.

5. Procédé suivant la revendication 4, dans lequel la région de boucle connectant les brins β B et E est randomisée.

6. Procédé suivant la revendication 4, dans lequel les régions de boucle connectant β-E et β-F, β-G et β-H et β-I et β-J sont randomisées.

7. Molécule d'ossature de protéine de liaison aux acides gras, désignée sous le nom de FASTbody, qui est dérivée d'une protéine intracellulaire eucaryotique pouvant être obtenue par le procédé de l'une quelconque des revendications 4 à 6.

8. Produit d'assemblage d'acide nucléique codant pour une molécule d'ossature de protéine de liaison aux acides gras, désignée sous le nom de FASTbody, suivant la revendication 1 ou la revendication 2.

9. Vecteur comprenant un produit d'assemblage d'acide nucléique suivant la revendication 8.

10. Cellule hôte comprenant un vecteur suivant la revendication 9.

11. Procédé pour modifier la spécificité de liaison de ligand d'une molécule d'ossature de liaison aux acides gras, désignée sous le nom de FASTbody, suivant l'une quelconque des revendications 1 à 7, procédé qui comprend les étapes consistant :
(a) à fournir une molécule d'ossature de liaison aux acides gras suivant la revendication 1 ou la revendication 2, et
(b) à randomiser une ou plusieurs boucles connectant les brins β appelés A, B, C, D, E, F, G, H, I et J.

12. Banque de molécules d'ossature de protéine de liaison aux acides gras, désignées sous le nom de FASTbodies, suivant la revendication 1 ou la revendication 2.

13. Procédé pour sélectionner une molécule d'ossature de liaison aux acides gras suivant l'une quelconque des revendications 1 à 7, molécule d'ossature qui est capable de se lier à un ligand défini, procédé qui comprend les étapes consistant :
(a) à fournir une banque de protéines de liaison aux acides gras, désignées sous le nom de FASTbody, suivant la revendication 12 ;
(b) à tester l'aptitude de la banque suivant l'étape (a) à se lier au ligand défini ; et
(c) à sélectionner les molécules d'ossature de liaison aux acides gras suivant l'étape (b) qui sont capables de se lier au ligand défini.

14. Utilisation d'un acide gras dans la modulation de la liaison d'un ligand spécifique à une ou plusieurs FASTbodies suivant l'une quelconque des revendications 1 et 2.

15. Utilisation d'un acide gras dans le contrôle de la liaison de ligands spécifiques à une ou plusieurs FASTbodies suivant l'une quelconque des revendications 1 et 2.

16. Procédé pour identifier un médicament candidat potentiel qui est capable de déplacer la liaison d'une FASTbody suivant l'une quelconque des revendications 1 à 7 à la cible, procédé qui comprend les étapes consistant :
(a) à fournir une banque de FASTbodies suivant la revendication 12 ;
(b) à fournir un échantillon d'un ou plusieurs médicaments candidats ; et
(c) à soumettre la banque à une sélection pour déterminer l'aptitude du ou des médicaments candidats à déplacer la liaison d'une ou plusieurs FASTbodies dans la banque suivant l'étape (a).
